# EUROPEAN PATENT APPLICATION

(11) **EP 4 566 531 A1**
(43) Date of publication of application: **11.06.2025**
(21) Application number: 24216308.7
(22) Date of filing: 28.11.2024
(51) Int. Cl.: A61B 5/055, A61B 5/369, A61B 5/00, G16H 30/40

(54) **SYSTEMS AND METHODS OF INTEGRATING ELECTROPHYSIOLOGICAL DATA WITH A VISUAL REPRESENTATION OF ASSOCIATED ELECTRODES AND CONTACTS IN A PATIENT-SPECIFIC THREE-DIMENSIONAL BRAIN MODEL**

(30) Priority: 28.11.2023 US 202363603470 P; 18.01.2024 US 202463622484 P; 27.11.2024 US 202418962378
(71) Applicant: Cadwell Laboratories, Inc., Richland, WA 99336 (US)
(72) Inventor: Cadwell, John, Richland, 99352 (US); Holub, Michal, 730707 Block 707 (SG); Barcus, Scott, Kennewick, 99337 (US); Bell, Robert, Couer d'Arlene, 83815 (US)
(74) Representative: Murgitroyd & Company

(57) **Abstract**

A method of enabling a user to visualize, review and analyze a patient's EEG data, pre-surgical data and post-surgical data, includes generating a unified viewing environment, wherein the unified viewing environment has at least a first view area and a second view area; importing, using the unified viewing environment, pre-surgical data and post-surgical data; acquiring EEG data of the patient; displaying, simultaneously, the EEG data in the first view area and the post-surgical data in the second view area of the unified viewing environment, wherein the EEG data is displayed as a vertical stack of a plurality of EEG traces, and wherein the post-surgical data is displayed without a need to pre-process and co-register with a three-dimensional coordinate system; and automatically associating visual representation of each of one or more electrodes and contacts in the post-surgical data with an EEG trace of the plurality of EEG traces.

## Description

### CROSS-REFERENCE

The present specification relies on United States Patent Provisional Application Number 63/622,484, titled "Systems and Methods of Integrating EEG Data with a Visual Representation of Associated Electrodes and Contacts in a Patient-Specific Three-Dimensional Brain Model" and filed on January 18, 2024, and United States Patent Provisional Application Number 63/603,470, of the same title and filed on November 28, 2023, and United States Patent Complete Application Number 18/962,378, of the same title and filed on November 27, 2024, for priority. The above-mentioned applications are herein incorporated by reference in their entirety.

### FIELD

The present specification is related generally to the field of diagnostic systems. More specifically, the present specification is related to integrating electrophysiological data with a visual representation of correlated electrodes and contacts in a patient-specific three-dimensional brain model supporting a plurality of analytical and visual functionalities.

### BACKGROUND

Brain surgery may be a treatment to stop epilepsy, reduce the number of epileptic seizures and/or decrease the severity of the epileptic seizures being experienced by a patient. Surgical approaches to manage epileptic seizures include removing portions of the patient's brain associated with the initiation of seizures, disconnecting brain nerve cell communication to stop the spread of seizures to other areas of the brain, using a laser to heat and kill nerve cells where seizures begin, implanting a pacemaker-like device and electrodes that send electrical signals to block or disrupt seizure activity at its source, and/or inserting delicate electrode wires (using robotic guidance) to record seizure activity from the depths of the patient's brain.

Healthcare providers perform pre-surgical testing on patients who are being considered for epilepsy surgery. There are usually two levels of pre-surgical testing. Phase I involves imaging and tests pre-surgery and phase II involves imaging and tests after a procedure for placing electrodes on the surface of the brain or within brain tissue.

Current pre-surgical clinical workflow identifies patients that are candidates for an intracranial EEG procedure. During this pre-surgical process, a plan of what regions of the brain are of importance to examine is defined and is executed by a neurosurgeon by placing different types of intracranial electrodes, both on the brain's surface (strip and grid electrodes) as well as within the brain tissue (sEEG electrodes). There are many different configurations of these intracranial electrodes having a large range of contacts, from a few (4) to many (>18), and many different configurations or parameters such as contact spacing, size, among others. A typical intracranial EEG recording can use 150-250 contacts in a single study. The neurosurgeon determines the most appropriate placement of these intracranial electrodes using different imaging techniques (MRI, CT, etc.) and different surgical tools (robots, surgical planning equipment) to place the intracranial electrodes.

Electrodes are surgically placed, and actual contact locations are recorded and may be verified with a CT scan. Thereafter, the EEG recording is started, whereby the electrophysiological activity (EEG) from the implanted electrodes is recorded. The EEG is typically displayed using different derivations (montages) as EEG traces (time series data).

Screen display constraints usually limit the number of displayed traces that can be effectively analyzed to less than 50. Also, multiple contacts on an individual electrode are usually sequential on the EEG trace display, but geometrically related contacts on different electrodes and anatomically proximate contacts are typically sparsely distributed on the long list of EEG traces. As such, the closest contacts may not be concurrently displayed on a single screen of EEG traces.

In addition, functional brain mapping may be conducted to recognize eloquent areas in the brain by applying electrical stimulation between selected electrode contacts chosen from the recording electrodes. The resulting EEG responses together with the impact it has on the patient, for instance motor twitches, sensory phenomena, visual impact etc. are observed and annotated. Significant responses include motor, sensory, visual, and perception as well as spreading brain activation detected by other contacts which can be normal, epilepsy-related or spontaneous. Each of these categories are manually noted for each contact.

The recording duration can range from a few to several days until sufficient data, typically a number of seizures with identifiable origin, has been recorded to decide on the clinical next step, such as determining whether brain resection is appropriate.

Specifically, it is currently difficult to tie what is seen in an EEG to the anatomy and symptoms of a patient. Existing systems do not combine a plurality of studies such as - 2D EEG tracing, pre and post-surgical imaging to provide a quick integrated analysis of brain pathology in a unified viewing environment - without the need for an expert to handle pre- and post-surgical imaging, co-registration (alignment and overlay of pre-surgical data from a single subject with that subject's own but separately acquired post-surgical data), and error correction.

Accordingly, there is need for systems and methods that enable correlating captured EEG traces with a visual representation of the electrodes (and specific contacts along the electrodes) associated with the captured EEG traces. There is also a need for the integration of EEG signals/traces with anatomical images in a unified viewing environment that a) enables a clinician to seamlessly transition between a three-dimensional model of the electrode contact locations within a patient's brain anatomy and a plurality of correlated EEG traces, and b) supports a plurality of functions and analyses for the clinician's use.

### SUMMARY

The following embodiments and aspects thereof are described and illustrated in conjunction with systems, tools and methods, which are meant to be exemplary and illustrative, and not limiting in scope. The present application discloses numerous embodiments.

In some embodiments, the present specification is directed towards a computer-implemented method of enabling a user to visualize EEG data in combination with pre-surgical data and post-surgical data using a computing device, wherein the computing device comprises at least one processor configured to execute programmatic instructions and a non-transient memory adapted to store the programmatic instructions, comprising: generating a first graphical user interface, wherein the first graphical user interface comprises at least a first display area and a second display area, wherein the first display area is separate and distinct from the second display area, and wherein the first and second display areas are positioned side-by-side; acquiring, using the computing device, the pre-surgical data and the post-surgical data, wherein the pre-surgical data comprises a three-dimensional first image of a patient's brain and comprises data indicative of a visual representation of at least one of an electrode trajectory and an electrode profile, wherein the post-surgical data comprises a three-dimensional second image of the patient's brain and comprises data indicative of a visual representation of at least one electrode surgically placed in the patient's brain, and wherein the three-dimensional first image and the three-dimensional second image are co-registered with a same three-dimensional coordinate system; acquiring the EEG data of the patient; displaying, simultaneously, the EEG data in the first display area and the post-surgical data in the second display area of the first graphical user interface, wherein the EEG data is displayed as a plurality of EEG traces; and automatically visually associating the visual representation of each of the at least one electrode in the post-surgical data with a corresponding one of the plurality of EEG traces.

Optionally, the method further comprises enabling a comparison of the post-surgical data with the pre-surgical data by highlighting, in the post-surgical data displayed in the second display area, one or more of the at least one electrode that deviates from the electrode trajectory by more than a predefined amount.

Optionally, the predefined amount is an offset percentage and wherein the offset percentage is in a range of 1% to 75%.

Optionally, the three-dimensional first image and the three-dimensional second image are at least one of a MRI image, a CT image, a SPECT image or a PET image.

Optionally, the at least one electrode comprises a plurality of electrodes and wherein the method further comprises: providing the user with a second graphical user interface adapted to receive a selection of one or more first contacts from a first one of the plurality of electrodes and a selection of one or more second contacts from a second one of the plurality of electrodes, wherein the first one of the plurality of electrodes and the second one of the plurality of electrodes are positioned respectively in a first anatomical region of the patient's brain and a second anatomical regions of the patient's brain and wherein the first anatomical region and the second anatomical region are different; and grouping said one or more first contacts and the one or more second contacts.

Optionally, the method further comprises providing the user with a second graphical user interface adapted to receive a selection of one or more EEG traces of the plurality of EEG traces, wherein the selected one or more EEG traces are indicative of the patient's potentially abnormal EEG activity; automatically highlighting, within the three-dimensional second image, one or more contacts of the at least one electrode that is associated with the selected one or more EEG traces; providing the user with a third graphical user interface adapted to receive a selection of at least one of the one or more contacts; providing the user with a fourth graphical user interface adapted to receive a specification of a region of interest around the selected at least one of the one or more contacts, wherein contacts of the at least one electrode that fall within the specified region of interest are automatically highlighted; and automatically generating an EEG montage of the region of interest using the contacts that are automatically highlighted within the specified region of interest.

Optionally, the highlighted contacts within the specified region of interest are physically associated with more than one electrodes of the at least one electrode. Optionally, the fourth graphical user interface is adapted to receive a geometrical shape, drawn by the user, around the selected electrode contact to specify the region of interest. Optionally, the fourth graphical user interface is adapted to retrieve data from an anatomical database to specify the region of interest.

Optionally, the method further comprises automatically identifying inoperable channels; and automatically highlighting the identified inoperable channels in the three-dimensional second image.

Optionally, the method further comprises automatically highlighting in the first display area one or more EEG traces of the plurality of EEG traces associated with the identified inoperable channels.

Optionally, the identified inoperable channels are associated with one or more of the at least one electrode positioned within white matter of the patient's brain. Optionally, the identified inoperable channels are associated with one or more of the at least one electrode located outside the patient's brain.

Optionally, the graphical user interface is configured to automatically generate a pop-up graphical user interface window when the user clicks an EEG trace of the plurality of EEG traces or causes a cursor to hover over said EEG trace, and wherein the pop-up graphical user interface window is configured to display the patient's brain anatomy that is centered proximate the at least one electrode associated with said EEG trace.

Optionally, the graphical user interface is configured to automatically generate a pop-up graphical user interface window when the user clicks a contact of the at least one electrode or causes a cursor to hover over said contact, and wherein the pop-up graphical user interface window is configured to display an EEG trace of the plurality of EEG traces associated with said contact.

Optionally, the method further comprises automatically generating data indicative of a degree of connectivity between two or more regions in the three-dimensional second image; and visually annotating said data indicative of the degree of connectivity in the three-dimensional second image.

Optionally, the visual annotation comprises one or more lines connecting the two or more regions. Optionally, the visual annotation comprises a color, different from a remainder of the three-dimensional second image, indicative of the degree of connectivity between the two or more regions.

Optionally, the method further comprises automatically highlighting one or more EEG traces of the plurality of EEG traces associated with the two or more regions.

Optionally, the method further comprises generating a second graphical user interface adapted to receive a selection of an anatomical space in the three-dimensional second image; and creating a label related to the anatomical space.

In some embodiments, the present specification is directed towards a system configured for enabling a user to visualize EEG data in combination with pre-surgical data and post-surgical data, comprising: an EEG system comprising a plurality of electrodes implanted into the patient's brain or spatially positioned on the patient's scalp, wherein the plurality of electrodes are in data communication with a multi-channel amplifier; and a computing device in data communication with the multi-channel amplifier and comprising: at least one processor and a non-volatile memory for storing programmatic code which, when executed, configures the processor to: generate a graphical user interface, wherein the graphical user interface comprises at least a first view area and a second view area, wherein the first view area is separate and distinct from the second view area, and wherein the first and second view areas are positioned side-by-side; acquire, using the computing device, the pre-surgical data and the post-surgical data, wherein the pre-surgical data comprises a MRI, CT, SPECT or PET based three-dimensional first image of the patient's brain, a visual representation of one or more planned electrode trajectories and electrode profiles, wherein the post-surgical data comprises a MRI, CT, SPECT or PET based three-dimensional second image of the patient's brain, wherein the second image includes a visual representation of one or more electrodes and contacts surgically placed in the patient's brain, and wherein the three-dimensional first image and the three-dimensional second image are co-registered with a three-dimensional coordinate system; acquire the EEG data of the patient; display, simultaneously, the EEG data in the first view area and the post-surgical data in the second view area of the graphical user interface, wherein the EEG data is displayed as a plurality of EEG traces; automatically associate the visual representation of each of one or more electrodes and contacts in the post-surgical data with corresponding one of the plurality of EEG traces; compare the post-surgical data with the pre-surgical data; and highlight, in the post-surgical data displayed in the second view area, one or more electrode contacts that deviate from their planned electrode trajectories by more than a predefined offset percentage.

Optionally, the predefined offset percentage is 1%, 5%, 10%, 15%, 20%, 25%, 30%, 50%, 75% or any increment therein.

Optionally, the system is further configured to enable the user to select one or more first electrode contacts from a first electrode and one or more second electrode contacts from a second electrode, wherein the first and second electrodes are positioned respectively in first and second anatomical regions of the patient's brain; and generating a group of said one or more first electrode contacts and said one or more second electrode contacts.

Optionally, the processor is further configured to enable the user to select one or more EEG traces of the plurality of traces, wherein the selected one or more EEG traces are indicative of potentially abnormal EEG activity; automatically highlight, within the three-dimensional second image, electrode contacts associated with the selected one or more EEG traces; enable the user to select an electrode contact from the highlighted electrode contacts; enable the user to specify a region of interest around the selected electrode contact, wherein electrode contacts that fall within the specified region of interest are automatically highlighted; and automatically generate a region of interest montage using the electrode contacts that are automatically highlighted within the specified region of interest.

Optionally, the highlighted electrode contacts that fall within the specified region of interest may not necessarily be related to a single electrode.

Optionally, the region of interest is specified by drawing a three-dimensional geometrical shape around the selected electrode contact.

Optionally, the region of interest is specified from an anatomical atlas.

Optionally, the processor is further configured to: automatically identify potentially bad channels; and automatically highlight the identified bad channels in the three-dimensional second image and one or more EEG traces associated with the identified bad channels.

Optionally, the identified bad channels are those positioned within white matter of the patient's brain. Optionally, the identified bad channels are located outside the patient's brain.

Optionally, the processor is further configured to: enable the user to click or hover mouse cursor over an EEG trace; and automatically generate a pop-up window over the EEG trace, wherein the pop-up window displays an anatomy centered around one or more electrode contacts associated with the EEG trace.

Optionally, the processor is further configured to: enable the user to click or hover mouse over an electrode contact displayed in the three-dimensional second image; and automatically generate a pop-up window over the electrode contact, wherein the pop-up window displays one or more EEG traces associated with the electrode contact.

Optionally, the processor is further configured to: automatically mark connectivity between two or more regions in the three-dimensional second image of the patient's brain; and automatically annotate results of brain connectivity analysis in the three-dimensional second image, wherein the connectivity analysis is performed on the two or more regions.

Optionally, the connectivity is marked with a line connecting the two or more regions. Optionally, the connectivity is marked by modulating color of the two or more regions.

Optionally, the processor is further configured to: automatically highlight one or more EEG traces associated with the two or more regions.

Optionally, the processor is further configured to: enable the user to select an arbitrary point within an anatomical space in the three-dimensional second model; and create a label related to the arbitrary point.

In some other embodiments, the present specification is directed towards a computer-implemented method of enabling a user to visualize and analyze a patient's EEG data, pre-surgical data and post-surgical data using a computing device, wherein the computing device comprises at least one processor configured to execute programmatic instructions and a non-transient memory adapted to store the programmatic instructions, comprising: generating a graphical user interface, wherein the graphical user interface comprises at least a first view area and a second view area, wherein the first view area is separate and distinct from the second view area, and wherein the first and second view areas are positioned side-by-side; acquiring, using the computing device, the pre-surgical data and the post-surgical data, wherein the pre-surgical data comprises a MRI, CT, SPECT or PET based three-dimensional first image of a patient's brain, a visual representation of one or more planned electrode trajectories and electrode profiles, wherein the post-surgical data comprises a MRI, CT, SPECT or PET based three-dimensional second image of the patient's brain, wherein the second image includes a visual representation of one or more electrodes and contacts surgically placed in the patient's brain, and wherein the three-dimensional first image and the three-dimensional second image are co-registered with a three-dimensional coordinate system; acquiring the EEG data of the patient; displaying, simultaneously, the EEG data in the first view area and the post-surgical data in the second view area of the graphical user interface, wherein the EEG data is displayed as a plurality of EEG traces; automatically associating the visual representation of each of one or more electrodes and contacts in the post-surgical data with corresponding one of the plurality of EEG traces; enabling the user to click or hover mouse over an EEG trace; and automatically generating a pop-up window over the EEG trace, wherein the pop-up window displays an anatomy centered around one or more electrode contacts associated with the EEG trace.

Optionally, the method further comprises enabling the user to click or hover mouse over an electrode contact displayed in the three-dimensional second model; and automatically generating a pop-up window over the electrode contact, wherein the pop-up window displays one or more EEG traces associated with the electrode contact.

Optionally, method further comprises automatically identifying potentially bad channels; and automatically highlighting the identified bad channels in the three-dimensional second image and one or more EEG traces associated with the identified bad channels.

Optionally, the identified bad channels are those positioned within white matter of the patient's brain. Optionally, the identified bad channels are located outside the patient's brain.

The present specification discloses a method for planning surgical placement of electrodes below the surface of a patient's brain and identifying areas of seizure activity, comprising: generating and displaying, in a first graphical user interface, a pre-surgical three-dimensional first model of the patient's brain, wherein the first model is aligned with a three-dimensional coordinate system; visualizing, using the first model, placement of one or more virtual electrodes in order to generate a baseline visual representation of placement of the one or more virtual electrodes; generating and displaying, in the first graphical user interface, a post-surgical three-dimensional second model of the patient's brain, wherein the second model is also aligned with said three-dimensional coordinate system, and wherein the second model enables a clinician to see actual visual representations of one or more electrodes surgically placed in the patient's brain; connecting each contact of the one or more electrodes to an input of an amplifier in order to capture a corresponding EEG trace; associating each EEG trace with a corresponding actual visual representation of each contact of the one or more electrodes in the second model; and generating a second graphical user interface to enable the clinician to transition between a first view area displaying vertically stacked EEG traces and a second view area displaying the actual visual representations of the associated one or more electrodes in the second model.

Optionally, the first model is generated by: imaging the patient using a magnetic resonance imaging (MRI) system in order to generate a three-dimensional MRI image; and processing the MRI image to at least one of remove background noise, adjust brightness levels or isolate an image of the patient's brain.

Optionally, the one or more virtual electrodes are selected from a library of manufacturer specific electrode profiles and manually positioned on the first model by the clinician, wherein the electrode profiles comprise at least one of a type of electrode, a physical dimension of the electrode, a number of contacts on each electrode, a serial number, a material composition, a manufacturing lot, a date of first use, or sterilization data.

Optionally, the second model is generated by: imaging the patient using a Computed Tomography (CT) system in order to generate a three-dimensional CT image; and processing the CT image to at least one of remove background noise, adjust brightness levels or isolate the patient's brain.

Optionally, the method further comprises: comparing the baseline visual representation in the first model with the actual visual representation in the second model; and visually highlighting at least one of the one or more electrodes that are off by more than a predefined offset percentage with respect to the baseline visual representation.

Optionally, the method further comprises automatically highlighting an EEG trace in the first view area when the clinician selects or hovers a mouse cursor over an electrode contact in the second view area, and automatically highlighting an electrode contract in the second view area when the clinician selects or hovers the mouse cursor over an EEG trace in the first view area.

The present specification also discloses a system for planning surgical placement of electrodes below the surface of a patient's brain and identifying areas of seizure activity, comprising: an EEG system having a plurality of electrodes implanted into the patient's brain that are in data communication with a multi-channel amplifier in order to capture EEG traces; a magnetic resonance imaging (MRI) system that generates a pre-surgical three-dimensional first model of the patient's brain; a computed tomography (CT) system that generates a post-surgical three-dimensional second model of the patient's brain; and a computing device in data communication with the EEG, MRI and CT systems and including a processor, display and a memory that stores a plurality of programmatic instructions which when executed cause the processor to: display, in a first graphical user interface, the first model and enable the clinician to align the first model with a three-dimensional coordinate system; enable the clinician to simulate, using the first model, placement of one or more virtual electrodes in order to generate a baseline visual representation of placement of the one or more virtual electrodes; display, in the first graphical user interface, the second model and enable the clinician to align the second model with the three-dimensional coordinate system, wherein the second model enables the clinician to see actual visual representations of one or more electrodes surgically placed in the patient's brain; associate each EEG trace with a corresponding actual visual representation of each contact of the one or more electrodes in the second model; and generate a second graphical user interface to enable the clinician to seamlessly transition between a first view area displaying vertically stacked EEG traces and a second view area displaying the actual visual representations of the associated one or more electrodes in the second model.

Optionally, the first model is processed to remove background noise, adjust brightness levels and isolate the patient's brain.

Optionally, the three-dimensional coordinate system has a resolution in each axis of 1mm and 256 sections in each axial direction with 0 in the center.

Optionally, the one or more virtual electrodes are selected from a library of manufacturer specific electrode profiles and manually positioned on the first model by the clinician, and wherein the electrode profiles include types of electrodes, physical dimensions of the electrodes, number of contacts in each of the electrodes, serial numbers, material compositions, manufacturing lots, dates of first use, and sterilization details.

Optionally, the second model is processed to remove background noise, adjust brightness levels and isolate the patient's brain.

Optionally, the processor is further caused to: compare the baseline visual representation in the first model with the actual visual representation in the second model; and visually highlight at least one of the one or more electrodes that are off by more than a predefined offset percentage with respect to the baseline visual representation.

Optionally, if the clinician selects or hovers a mouse cursor over an electrode contact in the second view area the associated EEG trace gets automatically highlighted in the first view area, and if the clinician selects or hovers the mouse cursor over an EEG trace in the first view area the associated contact gets automatically highlighted in the second view area.

Optionally, the processor is further caused to enable the clinician to select anode and cathode contacts in the second view area and apply a visual marker indicative of each contact being anode or cathode.

Optionally, the processor is further caused to enable the clinician to use a volume capture feature to select a group of electrode contacts in the second view area and automatically visualize which EEG traces in the first view area correlate with each of the selected electrode contacts.

The present specification also discloses a method for planning surgical placement of electrodes below the surface of a patient's brain and identifying areas of seizure activity, comprising: generating and displaying, in a first graphical user interface, a pre-surgical three-dimensional first model of the patient's brain, wherein the first model is aligned with a three-dimensional coordinate system; simulating, using the first model, placement of one or more virtual electrodes in order to generate a baseline visual representation of placement of the one or more virtual electrodes; generating and displaying, in the first graphical user interface, a post-surgical three-dimensional second model of the patient's brain, wherein the second model is also aligned with the three-dimensional coordinate system, and wherein the second model enables a clinician to see actual visual representations of one or more electrodes surgically placed in the patient's brain; connecting each contact of the one or more electrodes to an input of an amplifier in order to capture a corresponding EEG trace; associating each EEG trace with a corresponding actual visual representation of each contact of the one or more electrodes in the second model; generating a second graphical user interface to enable the clinician to seamlessly transition between a first view area displaying vertically stacked EEG traces and a second view area displaying the actual visual representations of the associated one or more electrodes in the second model; comparing the baseline visual representation in the first model with the actual visual representation in the second model; and visually highlighting at least one of the one or more electrodes that are off by more than a predefined offset percentage with respect to the baseline visual representation.

Optionally, the first model is generated by: imaging the patient using a magnetic resonance imaging (MRI) system in order to generate a three-dimensional MRI image; and processing the MRI image to remove background noise, adjust brightness levels and isolate the patient's brain.

Optionally, the second model is generated by: imaging the patient using a Computed Tomography (CT) system in order to generate a three-dimensional CT image; and processing the CT image to remove background noise, adjust brightness levels and isolate the patient's brain.

Optionally, the method further comprises enabling the clinician to use a volume capture feature to select a group of electrode contacts in the second view area and automatically visualize which EEG traces in the first view area correlate with each of the selected electrode contacts.

The aforementioned and other embodiments of the present specification shall be described in greater depth in the drawings and detailed description provided below.

### BRIEF DESCRIPTION OF THE DRAWINGS

The accompanying drawings illustrate various embodiments of systems, methods, and embodiments of various other aspects of the disclosure. Any person with ordinary skills in the art will appreciate that the illustrated element boundaries (e.g., boxes, groups of boxes, or other shapes) in the figures represent one example of the boundaries. It may be that in some examples one element may be designed as multiple elements or that multiple elements may be designed as one element. In some examples, an element shown as an internal component of one element may be implemented as an external component in another and vice versa. Furthermore, elements may not be drawn to scale. Non-limiting and non-exhaustive descriptions are described with reference to the following drawings. The components in the figures are not necessarily to scale, emphasis instead being placed upon illustrating principles.
FIG. 1 illustrates an integrated multi-modality system, in accordance with some embodiments of the present specification;
FIG. 2 is a flowchart of a plurality of steps of an integrated multi-modality exam workflow, in accordance with some embodiments of the present specification;
FIG. 3A shows a first unified viewing environment that displays EEG traces in association with a visual representation of corresponding electrodes and contacts in a patient's 3D brain model, in accordance with some embodiments of the present specification;
FIG. 3B shows another view of the first unified viewing environment of FIG. 3A, in accordance with some embodiments of the present specification;
FIG. 3C shows a second unified viewing environment that enables a clinician to visualize association of EEG and imaging data indicative of the patient's 3D brain model, in accordance with some embodiments of the present specification;
FIG. 3D shows another view of the second unified viewing environment of FIG. 3C, in accordance with some embodiments of the present specification;
FIG. 3E shows a third unified viewing environment that illustrates highlighting of all contacts and/or EEG traces with position in a current image plane, in accordance with some embodiments of the present specification;
FIG. 3F shows a fourth unified viewing environment that illustrates automatic modulation of the color palette of EEG traces in response to rotation of the 3D brain model, in accordance with some embodiments of the present specification;
FIG. 3G is an area of a viewing environment showing automatic creation of one or more extended bipolar montages, in accordance with some embodiments of the present specification;
FIG. 3H shows a fifth unified viewing environment that illustrates visualization of different aspects and quantitative properties of an EEG in a 3D brain model, in accordance with some embodiments of the present specification;
FIG. 3I shows a sixth unified viewing environment that illustrates functional result highlighting, in accordance with some embodiments of the present specification;
FIG. 3J is a seventh unified viewing environment that illustrates validation of contact positions for various applications using information gathered during intracranial stimulation, in accordance with some embodiments of the present specification;
FIG. 3K is an eighth unified viewing environment that illustrates highlighting of EEG traces and associated electrode contacts, in accordance with some embodiments of the present specification;
FIG. 3L shows a ninth unified viewing environment that illustrates an ROI montage displayed in a first view area, in accordance with some embodiments of the present specification;
FIG. 3M shows a tenth unified viewing environment that illustrates only a newly created ROI montage in a first view area, in accordance with some embodiments of the present specification;
FIG. 3N shows an eleventh unified viewing environment that illustrates an anatomical atlas within a 3D brain model, in accordance with some embodiments of the present specification;
FIG. 3O shows a twelfth unified viewing environment that illustrates visual indication of contact quality in a 3D brain model as well as in associated EEG traces, in accordance with some embodiments of the present specification;
FIG. 3P shows a thirteenth unified viewing environment that illustrates detected visual indication of contact quality in a 3D brain model as well as in associated EEG traces, in accordance with some embodiments of the present specification;
FIG. 3Q is an area of a viewing environment illustrating detection of quality of contacts based on their spatial location within a patient's brain, in accordance with some embodiments of the present specification;
FIG. 3R is an area of a viewing environment illustrating detection of quality of contacts based on their spatial location within and outside of a patient's brain, in accordance with some embodiments of the present specification;
FIG. 3S is a fourteenth unified viewing environment that illustrates an anatomy pop-up window displayed over an associated EEG trace, in accordance with some embodiments of the present specification;
FIG. 4 is a flowchart of a plurality of exemplary steps of a method for creating ROI montages, in accordance with some embodiments of the present specification; and
FIG. 5 illustrates an approach for individually addressing each contact within first and second electrodes positioned within a 3D brain model of a patient, in accordance with some embodiments of the present specification.

### DETAILED DESCRIPTION

The present specification discloses systems and methods for presenting co-registered pre and post-surgical data with intracranial electrode positions and their electrophysiological data in a unified viewing environment (also referred to as a 'graphical user interface (GUI)') that does not require advanced skill sets to generate, thereby enabling novel and improved clinical functionality. Historically, being able to work cohesively with such data has been the domain of practitioners having highly specialized skills using highly specialized tool sets and requiring significant time for each patient (on the order of days). Moreover, this approach is particularly prone to errors.

The systems and methods of the present specification make this domain available and able to be used by "normal" epileptologists and clinicians, by providing an automatic link between pre-surgical data, post-surgical data and typical EEG software. The unified viewing environment of the present specification enables a plurality of functionalities, such as, but not limited to: a) workflow improvements to the planning and execution of an intracranial procedure leading to faster and improved clinical workflow, clinical functionality, and patient interaction; b) simple (one-click) import of pre-processed and co-registered pre- and post-surgical data with intracranial electrode positions and their electrophysiological data supporting both entirely new, as well as highly improved clinical functionality, providing new clinical information and powerful visualization and analysis capabilities; c) automated identification of anatomical regions of interest (ROI) based on the integration of intracranial electrode position data with corresponding electrophysiological data, including automatic identification of noisy/bad channels based at least on their anatomical positions; and d) identification of brain region connectivity in relation to anatomical features.

The present specification is directed towards multiple embodiments. The following disclosure is provided in order to enable a person having ordinary skill in the art to practice the invention. Language used in this specification should not be interpreted as a general disavowal of any one specific embodiment or used to limit the claims beyond the meaning of the terms used therein. The general principles defined herein may be applied to other embodiments and applications without departing from the spirit and scope of the invention. Also, the terminology and phraseology used is for the purpose of describing exemplary embodiments and should not be considered limiting. Thus, the present invention is to be accorded the widest scope encompassing numerous alternatives, modifications and equivalents consistent with the principles and features disclosed. For purposes of clarity, details relating to technical material that is known in the technical fields related to the invention have not been described in detail so as not to unnecessarily obscure the present invention.

In various embodiments, a computing device includes an input/output controller, at least one communications interface and system memory. The system memory includes at least one random access memory (RAM) and at least one read-only memory (ROM). These elements are in communication with a central processing unit (CPU) to enable operation of the computing device. In various embodiments, the computing device may be a conventional standalone computer or alternatively, the functions of the computing device may be distributed across multiple computer systems and architectures.

In some embodiments, execution of a plurality of sequences of programmatic instructions or code enable or cause the CPU of the computing device to perform various functions and processes. In alternate embodiments, hard-wired circuitry may be used in place of, or in combination with, software instructions for implementation of the processes of systems and methods described in this application. Thus, the systems and methods described are not limited to any specific combination of hardware and software.

The term "module" used in this disclosure may refer to computer logic utilized to provide a desired functionality, service or operation by programming or controlling a general-purpose processor. Stated differently, in some embodiments, a module, application or engine implements a plurality of instructions or programmatic code to cause a general-purpose processor to perform one or more functions. In various embodiments, a module, application or engine can be implemented in hardware, firmware, software or any combination thereof. The module, application or engine may be interchangeably used with unit, logic, logical block, component, or circuit, for example. The module, application or engine may be the minimum unit, or part thereof, which performs one or more particular functions.

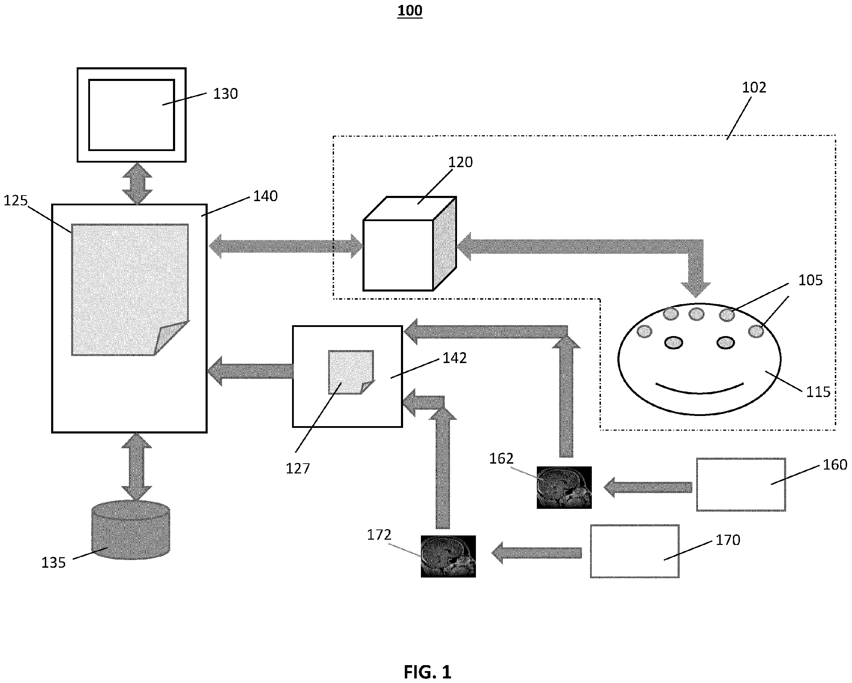

The term "average reference" as used in this disclosure may refer to a reference scheme where, for a given space around a selected contact, all electrical activity from contacts within the space is averaged and used as a reference for other contacts within the space. The space may be a sphere, cube, another standard geometric volume or a custom selected volume of interest. In some embodiments, a module (such as module 125 described with reference to FIG. 1 below) is configured to automatically choose electrodes to be included in the average based on their signal content. If there is too much activity on an electrode, then that specific signal may not be included in the average.

The term "weighted average reference" as used in this disclosure may refer to a reference scheme which is similar to "average reference" however, one or more of the signals are weighted relative to the other signals based on some metric, such as the proximity to a selected electrode.

The term "nearest neighbor reference" as used in this disclosure may refer to a reference scheme in which whichever contact is closest to a selected contact is used as a reference, regardless of which electrode it is part of.

The term "signal content reference" used in this disclosure may refer to a reference scheme in which a module (such as module 125 described with reference to FIG. 1 below) is configured to run through a plurality of different combinations of active and reference electrodes, analyze the results and thereafter select only those EEG traces that contain a signal of interest, automatically, based on its signal content.

The term "montage" used in this disclosure may refer to a logical arrangement of channels (a comparison of two EEG amplifier inputs) which convey information about electrical signals in brain.

The term "EEG trace" used in this disclosure may refer to the waveform of a montage channel resulting from a mathematical difference of two input waveforms.

The term "region of interest (ROI)" used in this disclosure may refer to regions of the brain identified in Phase 1 tests using a number of tests, including EEG, SPECT, fMRI, Wada, CT< or neuropsychological evaluation, as having a higher probability of abnormal, unwanted, and/or seizure activity. These regions are inferred from a dense set of scalp electrodes and a complex estimate based on tissue conduction and many other factors.

The term "marching cubes algorithm" used in this disclosure may refer to a computer graphics algorithm for extracting a polygonal mesh of an isosurface from a three-dimensional discrete scalar field (the elements of which are sometimes called voxels).

In the description and claims of the application, each of the words "comprise", "include", "have", "contain", and forms thereof, are not necessarily limited to members in a list with which the words may be associated. Thus, they are intended to be equivalent in meaning and be open-ended in that an item or items following any one of these words is not meant to be an exhaustive listing of such item or items, or meant to be limited to only the listed item or items. It should be noted herein that any feature or component described in association with a specific embodiment may be used and implemented with any other embodiment unless clearly indicated otherwise.

It must also be noted that as used herein and in the appended claims, the singular forms "a," "an," and "the" include plural references unless the context dictates otherwise. Although any systems and methods similar or equivalent to those described herein can be used in the practice or testing of embodiments of the present disclosure, the preferred, systems and methods are now described.

### Overview

FIG. 1 is a diagram illustrating an integrated multi-modality system 100, in accordance with some embodiments of the present specification. In various embodiments, the system 100 provides electroencephalography (EEG) and medical imaging data integration, viewing, manipulation and analyses capabilities. In some embodiments, the system 100 comprises a first modality corresponding to an EEG system 102 for detecting, diagnosing, or predicting neurological events from EEG signals. FIG. 1 shows a plurality of EEG sensors or electrodes 105 implanted into a patient's brain or spatially positioned on a layer of tissue such as the scalp of the patient 115. The plurality of electrodes 105 are in data communication with a multi-channel amplifier 120 that is in data communication with a first computing device 140. The first computing device 140 is in data communication with a display unit 130 and at least one database 135.

In various embodiments, the plurality of electrodes 105 include grid, strip and/or depth electrodes where each electrode may have multiple contacts. The plurality of electrodes 105 record electrical signals (EEG signals) from the patient's brain and communicate the analog signals over a first communication link to the multi-channel amplifier 120 that amplifies the signals, converts the signals from an analog EEG data set to a digital EEG data set, and communicates the resultant digital EEG data to the first computing device 140 over a second communication link.

In some embodiments, the system 100 comprises a second modality corresponding to a first imaging system 160 that generates and communicates patient-specific first image data 162 to a second computing device 142 over a third communication link and a third modality corresponding to an ionizing radiation based second medical imaging system 170 that generates and communicates patient-specific second image data 172 to the computing device 142 over a fourth communication link.

The second computing device 142 includes a third-party surgical planning software or application 127 such as, for example, ROSA (Robotic Stereotactic Assistance) that is used to generate processed pre- and post-surgical data. In accordance with some aspects of the present specification, the processed pre and post-surgical data is imported by a clinician into the first computing device 140, from the second computing device 142, over a fifth communication link. In embodiments, the first, second, third, fourth and fifth communication links may be dynamic, the same, different, wired and/or wireless.

In various embodiments, the image data generated by the first imaging system 160 and the second imaging system 170 may correspond to any of a plurality of imaging modalities such as, for example, Magnetic Resonance Imaging (MRI), Computed Tomography (CT), Single Proton Emission Camera Tomography (SPECT), or Positron Emission Tomography (PET) imaging. In various embodiments, each of the plurality of imaging modalities provide image data indicative of a series of images representing information in 3D space.

Each of the first and second computing devices 140, 142, respectively, include an input/output controller, at least one communications interface and system memory. The system memory includes at least one random access memory (RAM) and at least one read-only memory (ROM). These elements are in communication with a central processing unit (CPU) to enable operation of the first and second computing devices 140, 142 respectively. In various embodiments, each of the first and second computing devices 140, 142, respectively, may be a conventional standalone computer or alternatively, the functions of each of the first and second computing devices 140, 142, respectively, may be distributed across multiple computer systems and architectures. For example, in the case of the first computing device 140, in a distributed architecture the at least one database 135 and processing circuitry are housed in separate units or locations. Some units perform primary processing functions and contain at a minimum a general controller or a processing circuitry and a system memory.

### Pre- and post-surgical data

### Pre-surgical data

As mentioned earlier, the second computing device 142 includes a third-party surgical planning software or application 127 that is configured to process and enable co-registration of the patient-specific first image data 162 and second image data 172.

During a pre-surgical planning stage, the patient is first imaged using the second modality in order to generate the first image data 162. In some embodiments, the second modality corresponds to a magnetic resonance imaging (MRI) system and the first image data 162 corresponds to MRI image data indicative of a series of MRI images representing information in 3D space.

The MRI image data is received at the second computing device 142 and processed, using the third-party surgical planning software 127, to perform a plurality of functions such as, but not limited to, removing background noise, adjusting brightness levels, isolating the patient's brain (for example by selecting, either manually or by using an automated tool, the area of the brain and removing extracorporeal data), adjusting offsets and rotations to defined co-registered locations and aligning with a predefined or standard three-dimensional coordinate system. This processing yields processed and co-registered pre-surgery first image data 162 indicative of MRI-based 3D brain model (for example, a Cortical Surface Projection model - which is a 3D model of an outer surface of the patient's brain) of the patient.

Within the third-party surgical planning software 127, the MRI-based 3D brain model of the patient is used to further simulate placement of virtual electrodes thereby generating a surgical plan, a baseline or goal visual representation of electrode placements or trajectories including a trajectory list (that includes data indicative of dimensions of each electrode as well as where each electrode starts and ends spatially). That is, the 3D Cortical Surface Projection model is used to simulate where the electrodes should preferably be placed in the brain. This enables visualization of virtual electrodes representing the planned surgically placed electrodes within the MRI-based 3D brain model.

Typically, virtual electrode types are selected from a library of manufacturer specific electrode profiles and the selected electrodes are manually positioned on the model. Electrode profiles include information such as, but not limited to, type of electrode (grid and depth are the most common), physical dimensions of the electrode (allowing it to be scaled and rendered in the 3D model), number of contacts, serial number, material composition, manufacturing lot, date of first use, and/or sterilization details. Alternatively, ROSA (Robotic Stereotactic Assistance) trajectories, which are robot-assisted electrode placement trajectories, may be imported into the third-party surgical planning software 127. Subsequently, virtual electrodes are manually manipulated to match these trajectories.

Thus, the third-party surgical planning software 127 is used to generate pre-surgical data indicative of a) processed and co-registered pre-surgery first image data 162 indicative of MRI-based 3D brain model, and b) a surgical plan, a baseline or goal visual representation of electrode placements or trajectories including a trajectory list and electrode profiles. In various embodiments, the first image data 162 is at least one of MRI, CT, SPECT or PET based 3D brain model.

### Post-surgical data

After surgically implanting or positioning electrodes in the patient's brain, an accurate patient-specific anatomical 3D model is generated, incorporating a visual representation of the location, position, orientation, and dimensionality of the implanted or positioned electrodes. To do so, the patient is imaged using the third modality in order to generate the second image data 172. In some embodiments, the third modality corresponds to a CT system and the second image data 172 corresponds to CT image data indicative of a series of CT images representing information in 3D space.

The CT image data is received at the computing device 142 and processed, using the third-party surgical planning software 127, to perform a plurality of functions such as, but not limited to, removing background noise, adjusting brightness levels, isolating the patient's brain (for example by selecting, either manually or by using an automated tool, the area of the brain and removing extracorporeal data), and aligning with the same coordinate system as the first image data 162. This processing yields post-surgical data indicative of processed and co-registered post-surgery second image data 172 indicative of CT-based 3D brain model of the patient. In various embodiments, the second image data 172 is at least one of MRI, CT, SPECT or PET based 3D brain model.

### Multi- modality exam module or engine 125

In accordance with aspects of the present specification, the first computing device 140 includes a multi-modality exam module or engine 125 that executes a plurality of instructions of programmatic code thereby configuring the module 125 to implement an integrated multi-modality exam workflow in order to record, display, analyze, and review data from the first exam modality 102, second exam modality 160, and third exam modality 170 as integrated multi-modal data.

Any steps not explicitly limited to a tangible, specifically designed hardware embodiment may be performed by a plurality of programming code being executed by a general processing computing device. In some embodiments, execution of sequences of programmatic instructions enable or cause the CPU to perform various functions and processes. In alternate embodiments, hard-wired circuitry may be used in place of, or in combination with, software instructions for implementation of the processes of systems and methods described in this application. Thus, the systems and methods described are not limited to any specific combination of hardware and software.

### An integrated multi-modality exam workflow

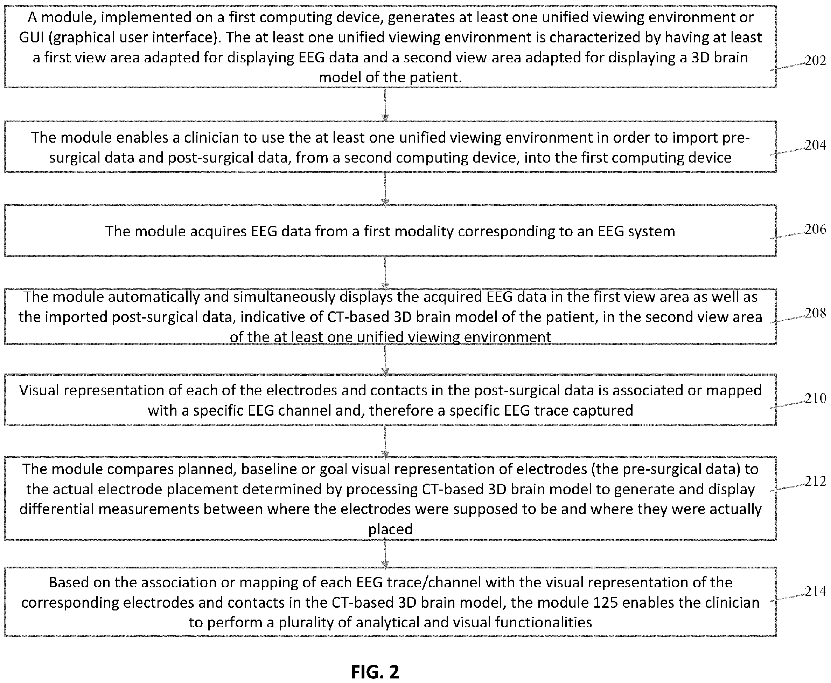

FIG. 2 is a flowchart of a plurality of steps of an integrated multi-modality exam workflow 200, in accordance with some embodiments of the present specification. In embodiments, the module 125 is configured to enable execution of the integrated multi-modality exam workflow 200.

Referring now to FIGS. 1 and 2, at step 202, the configured module 125 generates at least one unified viewing environment or GUI (graphical user interface). The at least one unified viewing environment is characterized by having at least a first view area adapted for displaying EEG data and a second view area adapted for displaying a 3D brain model of the patient. In some embodiments, the first view area is separate and distinct from the second view area (that is, they do not overlap). In some embodiments, the first and second view areas are positioned side-by-side (that is, adjacent to each other).

At step 204, the configured module 125 enables a clinician to use the at least one unified viewing environment in order to import pre-surgical data and post-surgical data, from the second computing device 142, into the first computing device 140. In some embodiments, the pre-surgical data represents a) processed and co-registered pre-surgery first image data 162 indicative of MRI-based 3D brain model of the patient, and b) a surgical plan, a baseline or goal visual representation of electrode placements or trajectories including a trajectory list and electrode profiles. In some embodiments, the post-surgical data represents processed and co-registered post-surgery second image data 172 indicative of CT-based 3D brain model of the patient.

It should be appreciated that, in accordance with advantages of the present specification, the clinician does not need to pre-process as well as co-register the first image data 162, simulate generation of pre-surgical electrode trajectories and pre-process as well as co-register the second image data 172 within the unified viewing environment. With a simple one-click import, the clinician brings the pre and post-surgical data from a third-party surgical planning software into the unified viewing environment. The import of the pre and post-surgical data ensures that there is no extra work needed by the clinician from the normal clinical workflow (since a manual co-registration of the MRI and CT-based 3D brain models along with planning electrode trajectories is a very time consuming and error prone process).

Thus, the module 125 enables input of pre and post-surgical data in a manner that requires no training on the part of the clinician beyond the act of importing external files. In particular, the clinician does not have to have advanced knowledge of brain physiology mapping. Thus, "normal" epileptologists and technicians can perform the import, enhancing their clinical experience and not be required to do anything other than import the pre and post-surgical data. More specifically, the clinician does not have to perform complicated co-registration and error analysis with respect to the pre and post-surgical data.

At step 206, the module 125 acquires EEG data from the first modality corresponding to the EEG system 102. To enable acquisition of the EEG data, each electrode contact is connected to an input of the amplifier 120 and one or more montages of channels of interest are created.

At step 208, the module 125 automatically and simultaneously displays the acquired EEG data in the first view area and the imported post-surgical data, indicative of CT-based 3D brain model of the patient, in the second view area of the at least one unified viewing environment. In some embodiments, the EEG data is displayed as a vertical stack of a plurality of EEG traces or waveforms.

At step 210, a visual representation of each of the electrodes and contacts in the post-surgical data is associated or mapped with a specific EEG channel and, therefore a specific EEG trace captured. This association or mapping, which may be achieved manually or automatically by the system, allows the clinician to easily select and track the electrode contacts to stimulate and/or use as a reference and visually see which traces correspond to what portions of the patient's anatomy and vice-versa. To enable the association, the data from each EEG trace/channel is correlated with the visual representation of a contact in the CT-based 3D brain model.

### Electrodes and contacts association or mapping with specific EEG traces

In some embodiments, virtual electrodes and contacts are selected from a library of manufacturer specific electrode profiles and matched (mapped) to the planned imported trajectories (from the pre-surgical data) within the CT-based 3D brain model.

In some embodiments, virtual electrodes and/or contacts are manually or automatically adjusted (by the module 125 configured to do so) to precisely match the metal electrodes and/or contacts visible in the CT scan within the CT-based 3D brain model thereby simplifying the process of mapping between the planned surgical trajectories and the actual placement of electrodes within the patient's brain.

It should be appreciated that the CT-based 3D brain model enables the clinician to visualize the electrode contacts surgically placed in the patient's brain. The module 125 uses known information such as the types and geometry of electrodes (that is, electrode profiles) being used to associate specific pixels with a contact and thereby precisely determine where each contact is located in the patient's brain. Stated differently, by receiving data defining the actual electrode profile, the model can discern the dimensionality of each electrode and relative distribution of contacts along each electrode and, using that information, determine that a particular set of pixels should be contacts, given their respective size and relative spacing, while a spurious pixel is unassociated with any electrode.

At step 212, the module 125 compares the planned, baseline or goal visual representation of electrodes (the pre-surgical data) to the actual electrode placement (the post-surgical data) determined by processing the CT-based 3D brain model to generate and display differential measurements or deviations between where the electrodes were supposed to be and where they were actually placed.

Actual placement of electrodes as visualized in the post-surgical CT-based 3D brain model differ from the intended electrode placement (the planned, baseline or goal visual representation of electrodes) due to any combination of the following reasons: the surgeons may change their minds during surgery, due to slight errors in robotic electrode placement electrodes may get implanted a "mm" or so away from the intended location and since the electrodes encounter pressures of the brain, although the entry and target may be where expected, the middle electrodes could flex deviating from a linear trajectory.

In some embodiments, the module 125 visually displays, in the unified viewing environment, the planned visual representation of electrodes versus actual trajectories of the electrodes. In some embodiments, the module 125 visually highlights, in the unified viewing environment relative to at least one of the MRI or CT brain model, differential placement of contacts that are off (or deviate) by more than a predefined offset percentage such as, for example, above 1%, above 5%, above 10%, above 15%, above 20%, above 25%, above 30%, above 50%, above 75% or any increment therein. Accordingly, the differential image demonstrates where, relative to the pre or post-surgical image, the projected electrodes or implanted electrodes were actually placed thereby minimizing errors in ascribing EEG activity to specific patient anatomical regions.

At step 214, based on the association or mapping of each EEG trace/channel with the visual representation of the corresponding electrodes and contacts in the CT-based 3D brain model, the module 125 enables the clinician to use the unified viewing environment to a) seamlessly transition between the first view area displaying vertically stacked EEG traces and the second view area displaying visual representations of locations of associated electrodes and contacts in the CT-based and/or MRI-based 3D brain model of the patient, and b) utilize and/or perform a plurality of analytical and visual functionalities.

### Unified Viewing Environment and Analytical and Visual Functionalities

As discussed earlier, in various embodiments, the multi-modality exam module 125 executes a plurality of instructions of programmatic code thereby configuring the module 125 to generate the at least one unified viewing environment, enable a plurality of analytical and visual functionalities for the clinician's use, enable the clinician to interact with the at least one unified viewing environment and manipulate data displayed in the at least one unified viewing environment.

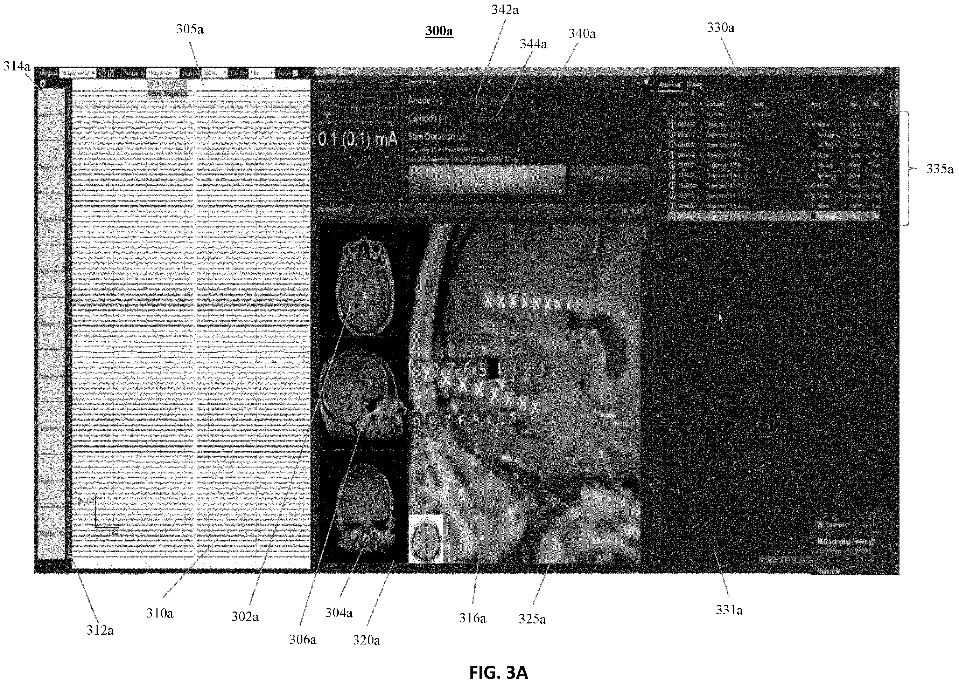

FIG. 3A shows a unified viewing environment 300a displaying EEG traces in association with a visual representation of corresponding electrodes and contacts in a patient's 3D brain model, in accordance with some embodiments of the present specification. In embodiments, the module 125 is configured to generate the unified viewing environment 300a and provide a plurality of functionalities for use by a clinician. The unified viewing environment 300a comprises a first view area 305a to display a plurality of vertically stacked EEG traces 310a along with corresponding electrodes 312a and trajectories 314a, a second view area 320a to display visual representations of locations of a plurality of electrodes and contacts in a 3D brain model 325a (the brain model may be the 3D CT based image co-registered with surgically placed electrodes or the 3D MRI based image co-registered with planned electrode trajectories) of the patient, a third view area 330a to display a list 335a of the plurality of electrode contacts and stimulation response characteristics of the patient with reference to each of the plurality of electrode contacts, and a fourth view area 340a to display a first drop-down list 342a to select an electrode contact as an anode from the plurality of electrode contacts and a second drop-down list 344a to select an electrode contact as a cathode from the plurality of electrode contacts. The second view area 320a also displays first, second and third image views 302a, 304a, 306a of the patient's 3D brain model. In the 3D brain model 325a, various numbers represent the plurality of electrode contacts.

In some embodiments, the first view area 305a is separate and distinct from the second view area 320a (that is, they do not overlap). In some embodiments, the first view area 305a and the second view area 320a are positioned side-by-side (that is, are positioned adjacent to each other).

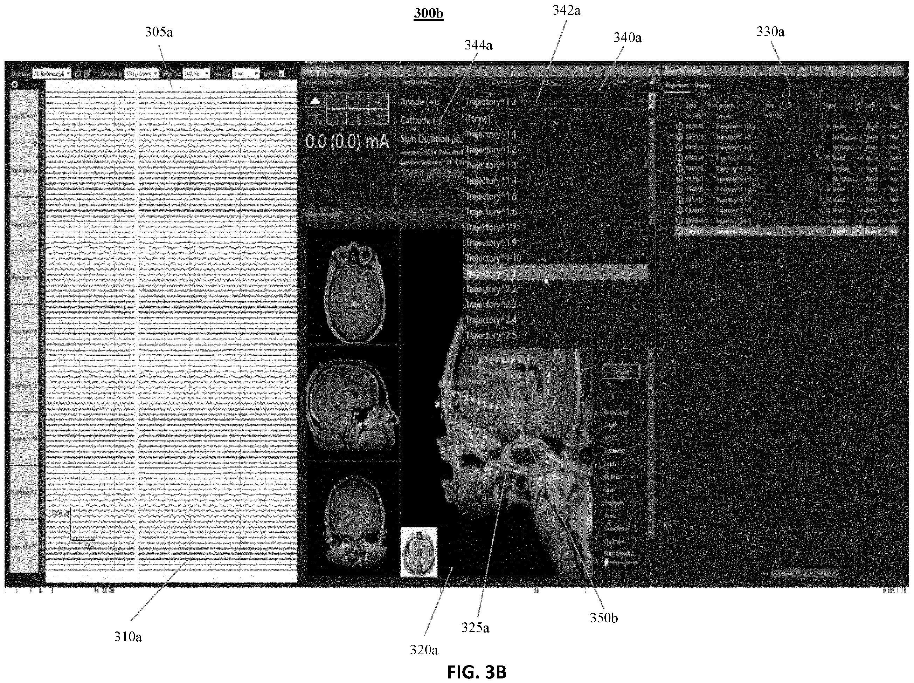

Referring now to FIG. 3A, in various embodiments, some of the plurality of analytical and visual functionalities that may be supported by the one or more unified viewing environments, such as at least the unified viewing environment 300a, are described below.
A) Electrodes and contacts can be visualized, within the 3D brain model 325a, as relative to one another, or repositioned, or added, or removed. Electrodes may be repositioned to reflect additions or changes during additional surgery. In some embodiments, the clinician may manipulate the virtual electrodes in the MRI-based 3D brain model to match the actual placement locations of the electrodes in the CT-based 3D brain model (which, in some embodiments, may be filtered to contrast electrodes only).
B) As shown in FIG. 3A and the unified viewing environment 300b of FIG. 3B, once a first electrode contact is selected, in the first drop-down list 342a (as an anode) and a second electrode contact is selected, in the second drop-down list 344a (as a cathode) - the visual representations 350b of the first electrode contact (anode) and second electrode contact (cathode) are automatically highlighted in the 3D brain model 325a (in the second view area 320a). Alternatively, if the visual representations of the first and second electrode contacts are selected/highlighted (such as, for example, by mouse clicks) as an anode and cathode, respectively, by the clinician in the 3D brain model 325a, the first and second electrodes are automatically populated as anode and cathode in the respective first and second drop-down lists 342a, 344a. Once selected, the system automatically initiates a stimulation using the selected anode/cathode and the selected mode of operation (for example, bipolar and referential where all electrodes reference to a single contact).
C) In some embodiments, the system supports the creation of new types of montages and references based on geographic proximity. The system also supports the creation and use of any type of reference (such as a specific electrode contact as a reference, average reference, weighted average reference, nearest neighbor reference, or signal content reference). The system captures the EEG traces based on the stimulation settings. Conventionally, a clinician would need to choose an alphanumeric description of the electrode (i.e., a name) from drop down menus and the clinician had to know to what part of the anatomy the electrode maps. The clinician is supported to observe and record stimulation responses on the electrode contacts in the form of EEG waveforms in the first view area 305a as well as in the form of a plurality of annotation categories of responses such as, but not limited to, motor response, sensory response, perception, and any other information in the third view area 330a. In some embodiments, the annotation categories are color-coded and can be sorted and filtered by the clinician. The third view area 330a displays all occurrences of the plurality of annotation categories of responses (motor, sensory, visual, perceptive, or similar information) visually. Selecting one or more of the plurality of annotation categories of responses, in the third view area 330a, automatically selects/highlights all corresponding electrode contacts.
D) In some embodiments, the module 125 is configured to enable the clinician to use the unified viewing environment 300a to uniquely group electrode contacts by anatomical region or brain function, as opposed to grouping contacts based on their physical relationship to each other. More specifically, a contact (or small metallic surface) is a single point of recording and associated with a single channel. Contacts are mechanically/physically connected with one another linearly (to form a vertical electrode) or in a grid fashion (to form a grid electrode). Conventionally, all mechanically associated contacts are grouped together in a single hardware group.

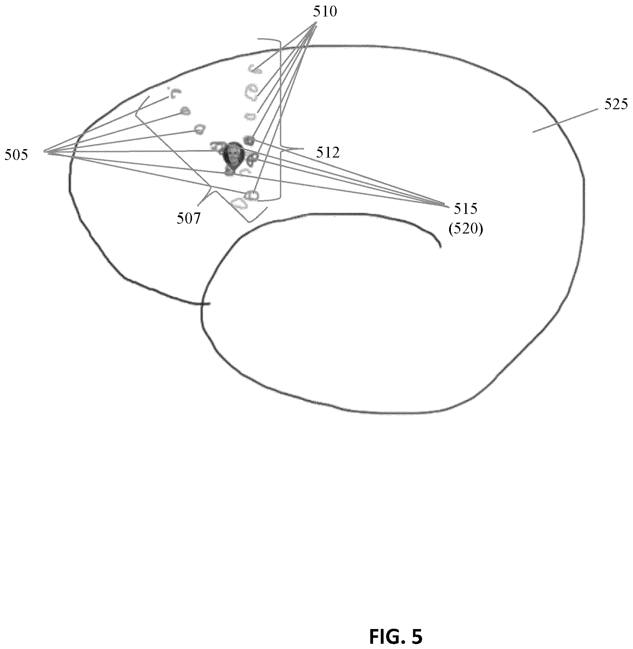

As shown in FIG. 5, a first plurality of contacts 505 are grouped together as a first single electrode 507 while a second plurality of contacts 510 are separately grouped together as a second single electrode 512. The first electrode 507 and second electrode 512 are shown positioned in a 3D brain model 525. In accordance with some aspects of the present specification, the module 125 is configured to treat each of the contacts of the respective plurality of contacts individually and independently of other contacts on the same electrode. Therefore, in some embodiments, the clinician can group together contacts from one electrode (for example, one or more of the first plurality of contacts 505 of first electrode 507) and contacts from another electrode (for example, one or more of the second plurality of contacts 510 of second electrode 512) by using a dialog box to input the names of the contacts or by touching/selecting the visual representations of the contacts on the screen, thus creating a new group 520 comprising such as, for example, a third plurality of contacts 515 (where the third plurality of contacts may include one or more of the first plurality of contacts 505 and one or more of the second plurality of contacts 510, in embodiments).

The new group 520, that is, the third plurality of contacts 515 may be based on anatomical region, their position in gray matter, their position in white matter, or their location in a region of the brain associated with a specific function. In various embodiments, once grouped, the channels associated with those contacts (that is, the third plurality of contacts 515) may then be shown separate from the other channels, either in a new graphical user interface (GUI) window or in a defined section. For example, there may be a separate portion 331a to display the channels associated with the third plurality of contacts 515 or there may be a separate portion within the existing portion 305a that is dedicated to displaying the channels associated with the third plurality of contacts 515. This ability to treat contacts independent of the hardware group or electrode with which the contact would otherwise be associated is uniquely advantageous to the clinician.

E) In some embodiments, the module 125 is configured to afford the clinician the use of the unified viewing environment 300a to select anode and cathode contacts in the 3D brain model 325a and apply a visual marker or annotation (textual and/or in the form of a graphical visual element or icon) to indicate differentiate the two.

F) In some embodiments, the module 125 is configured to, through the unified viewing environment 300a, suggest protocols for stimulation and define associated montages to record to evaluate a region of interest (ROI) allowing the clinician to optimize parameters. In some embodiments, these profiles or montages are created automatically during the surgical planning stage. Once surgery begins, a first stimulation site is already selected, waiting for the clinician to initiate stimulation. Also, a corresponding montage with the geographically selected contacts is displayed to capture responses.

G) In some embodiments, if the clinician clicks on an EEG trace 310a, the module 125 is configured to center the 3D brain model 325a around the contact(s) associated with the EEG trace 310a.

H) In some embodiments, if the clinician clicks on a contact 316a, displayed in the 3D brain model 325a, the module 125 is configured to center the three planes of imaging data indicative of the 3D brain model 325a on the contact 316a that has been clicked on.

I) In some embodiments, the module 125 is configured to enable the clinician to click any arbitrary point within the anatomical space in the 3D brain model 325a and create a label or annotation (textual and/or in the form of a graphical visual element or icon) related to or based on the clicked 3D location or arbitrary point. In some embodiments, the labeling or annotation is based on precise electrode-to-anatomy features. In various embodiments, the created labels and annotations enable the module 125 to implement a plurality of AI (Artificial Intelligence) based functions - such as, for example, automatic identification of labeled regions and automatic identification of epileptic zones.

J) In some embodiments, the module 125 is configured to enable the clinician to select EEG traces, in the first view area 305a, and visualize key spikes in the 3D brain model 325a as they happened over time, such as a propagation movie. The propagation movie shows electrical activity of interest (for example, a spike, high frequency oscillations(HFO), or seizure) movement through the 3D brain model 325a over time. The clinician may use this to perform spike detection and localization on individual spikes by generating vectors with location and direction. The module 125 is configured to display these vectors within the 3D brain model 325c to visualize clustering of spikes. Noise that is detected as a spike (false positive) will not tend to cluster, allowing visual filtering of true positives from false positives. The clustered sets can be selected using bubble selection feature for further analysis. Conventionally, a clinician only sees seizure activity in EEG traces and cannot really visualize what is actually happening in space. The unified viewing environment 300a enables the clinician to see the seizure spread out over all of the EEG traces.

The clinician is also enabled to select a region of interest (ROI) and then perform a frequency analysis over a period of time. The clinician is enabled to visualize how the selected EEG traces move in time. The clinician may also visualize how the frequencies change. Contacts share attributes and parameters such as, for example, frequency (how much, peak frequency, phase), amplitude or power, presence of high frequency components. These can be in absolute or relative values or as z-scores. Over multiple contacts, the 3D 'fields' of these types of analytical values are used to generate a volumetric representation of such values.

In some embodiments, a 3D version similar to a topographic map generates nested volumes, similar to babushka dolls, each of which describes a certain level of the field amplitude. Similarly, intracranial evoked potentials, especially from electrical stimulation of a contact, show neural propagation of the resulting response. The visualization of presumed cortical pathways/tracts generating the responses is supported by plotting the latency of the results across the array of contacts. The unified viewing environment 300a enables this to be visualized by the clinician in a movie format resembling a slow-motion explosion propagating from the stimulated contact. In some embodiments, the responses may be averaged across multiple stimuli to improve the signal to noise ratio.

K) Responses to a stimulus may appear over time on different channels (similar to ripple effect). In some embodiments, the module 125 is configured to display these responses over time on the 3D anatomical model 325a to visualize their physical distribution and direction over time.

L) In some embodiments, the module 125 is configured to enable fields (spheres) of response to be drawn (using a "marching cubes" algorithm) and combined to form new, more accurate ROIs (regions of interest) generated from actual electrical response.

M) In some embodiments, the module 125 is configured to indicate, within the unified viewing environment 300a, which contacts have already had a stimulation applied at them (for example, using a visual yellow tag).

### Visual Association of EEG and Imaging Data Representing the Patient's 3D Brain Model

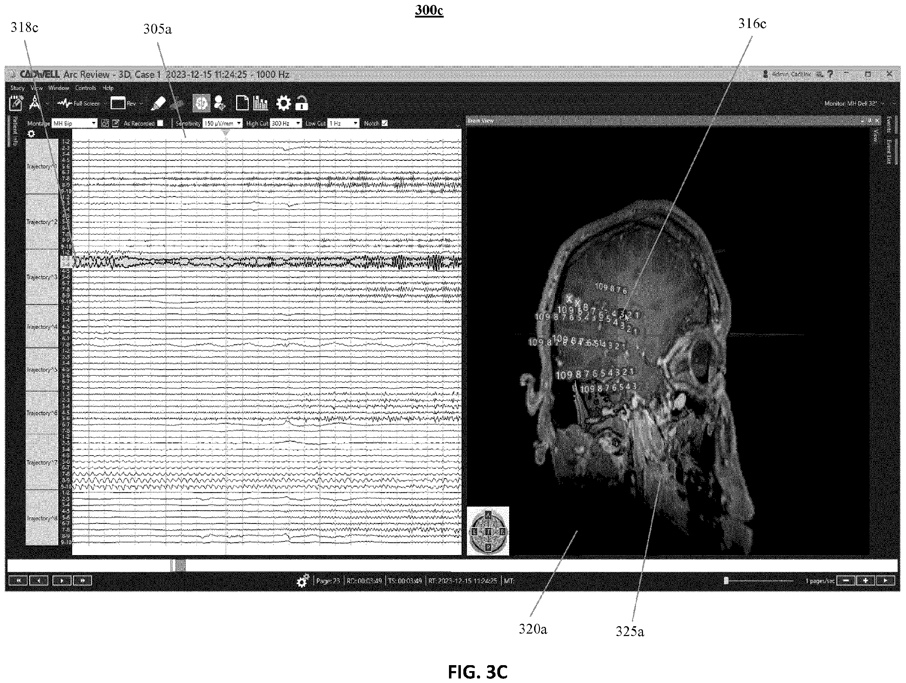

The association of each EEG trace/channel with the visual representation of the corresponding electrodes and contacts in the patient's 3D brain model allows the clinician to easily detect and visualize association of an EEG trace/channel with its anatomical origin in the patient's brain and vice versa. As shown in the unified viewing environment 300c of FIG. 3C, if the clinician selects (mouse-clicks), points, hovers or moves a mouse cursor over an electrode contact 316c in the 3D brain model 325a displayed in the second view area 320a, the associated one or more EEG traces 318c, corresponding to the electrode contact 316a, are automatically highlighted in the first view area 305a. Also, if the clinician selects (mouse-clicks), hovers or moves a mouse cursor over an electrode in the 3D brain model 325a, all associated EEG traces, corresponding to the contacts of the electrode, are automatically highlighted in the first view area 305a.

In some embodiments, the clinician can use a volume capture feature (for example a lasso tool or other selection tool) to select a group of electrode contacts in the patient-specific 3D brain model 325a. Once selected, the clinician automatically sees which EEG traces, in the first view area 305a, correlate with each of the selected electrode contacts. Close anatomical areas have traces that are highlighted together but the actual associated EEG traces may be far apart in the first view area 305a. In some embodiments, the lasso tool is a sphere but, in various embodiments, it can be any size or geometry. Therefore, if there is a volume in the brain that the clinician suspects are associated with seizure, then the unified viewing environment 300c enables the clinician to easily look at just the contacts in that brain volume. The clinician can select a target contact in the 3D brain model 325a and describe a contact around it. This is referred to as the bubble selection/capture volume and will show the associated EEG traces in the first view area 305a that are in that brain volume. The clinician may then select a contact in the 3D brain model 325a and (via bubble selection, for example) automatically select geographically close neighbors across multiple electrodes. This selection may be customized to reposition, add or remove contacts. This selection may be further used to filter the EEG montage to show only selected EEG traces of interest.

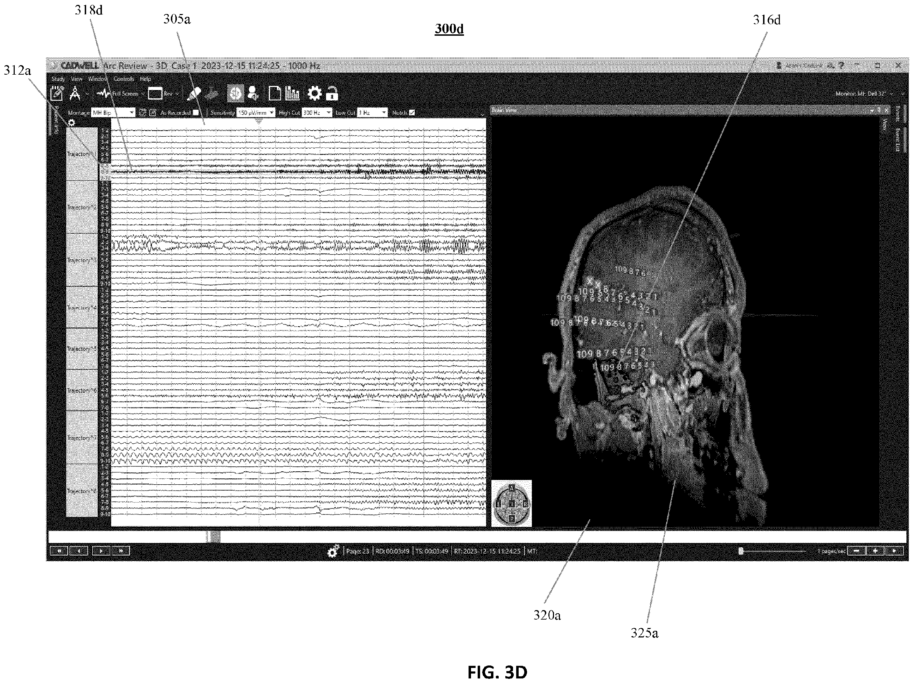

Similarly, as shown in the unified viewing environment 300d of FIG. 3D, if the clinician selects (mouse-clicks), hovers or moves the mouse cursor over an EEG trace 318d in the first view area 305a, the associated one or more electrode contacts 316d are automatically highlighted in the 3D brain model 325a in the second view area 320a. Again, if the clinician selects (mouse-clicks), hovers or moves the mouse cursor over an electrode block 312a in the first view area 305a, the associated electrode with all of its contacts is automatically highlighted in the 3D brain model 325a in the second view area 320a.

Further, if the clinician selects multiple EEG traces (for instance by using Ctrl+click) which may or may not be contiguous, in the first view area 305a, the associated electrode contacts are automatically highlighted in the 3D brain model 325a in the second view area 320a. Stated differently, the clinician can use a multi-trace selection tool to select multiple EEG traces vertically stacked atop each other in the first view area 305a. In embodiments, the selected EEG traces need not be vertically contiguous and may have a substantial degree of separation. Once selected, the clinician automatically sees, in the 3D brain model 325a, which electrode contracts correlate with each of the selected EEG traces. So, if there are multiple EEG traces of interest the clinician can see how they correlate to the regions of the patient's brain through the corresponding electrode contacts. Subsequently, the clinician can perform one or more analyses of the visual representations of the correlated electrode contacts.

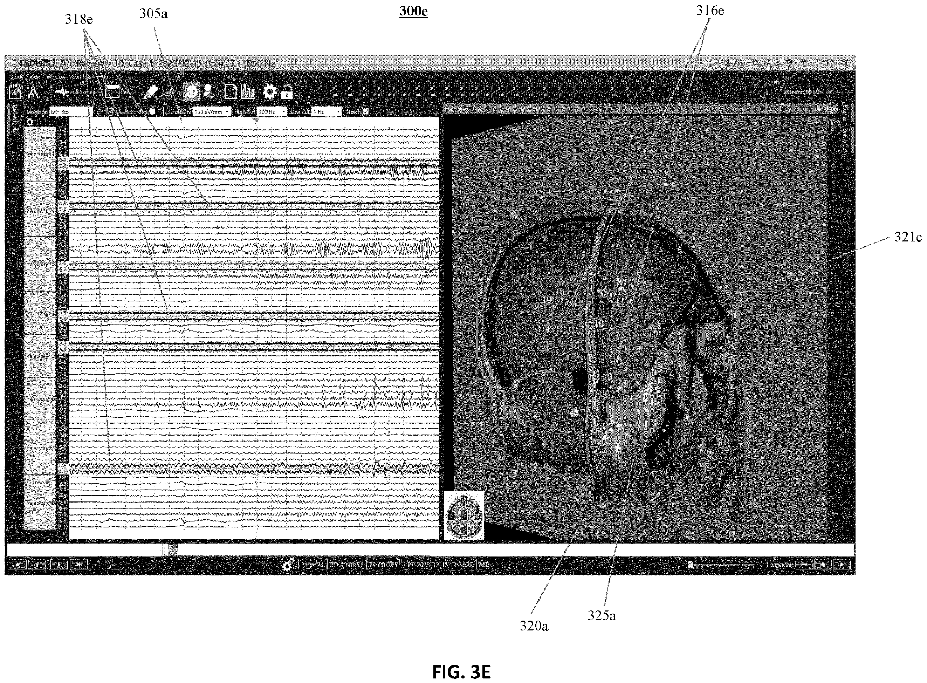

In some embodiments, the multi-modality exam module 125 is configured to use the unified viewing environment in order to highlight contacts and/or EEG traces in a current plane position. FIG. 3E is a unified viewing environment 300e that shows highlighting of all contacts and/or EEG traces having a position in a current image plane, in accordance with some embodiments of the present specification. In response to the clinician moving image slices of the 3D brain model 325a (in the second view area 320a), the module 125 automatically highlights all electrode contacts 316e (in the second view area 320a) and/or traces 318e (in the first view area 305a) having a position in the current image plane 321e.

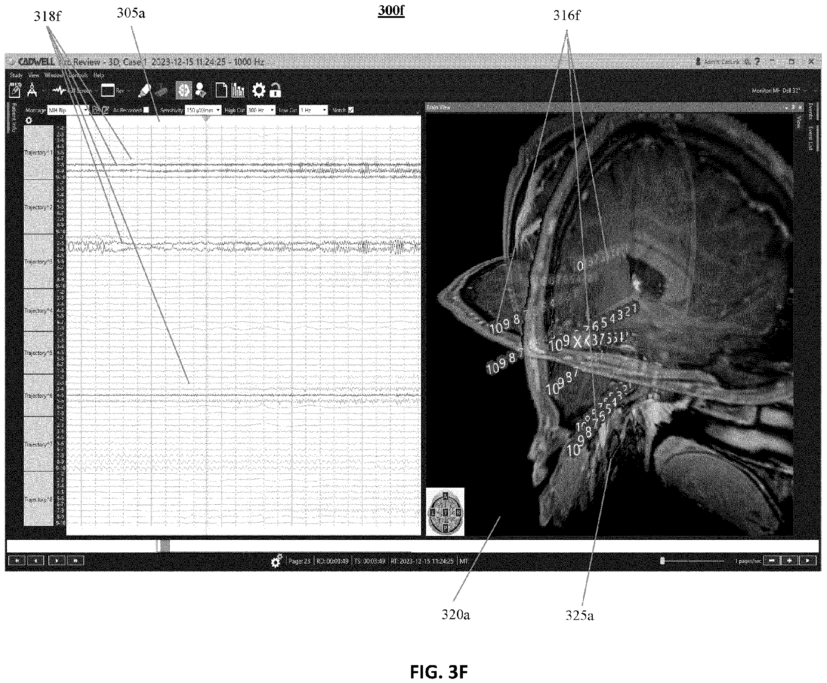

In some embodiments, the multi-modality exam module 125 is configured to use the unified viewing environment in order to automatically modulate a visual appearance of EEG traces in accordance with a distance of associated electrode contacts from a particular viewpoint. In some embodiments, the viewpoint can be automatically set based on a current view or placed at a point in space. FIG. 3F is a unified viewing environment 300f that shows automatic modulation of the color palette of the EEG traces in response to rotation of the 3D brain model, in accordance with some embodiments of the present specification. In response to the clinician rotating the 3D brain model 325a (in the second view area 320a), the module 125 automatically modulates/adjusts the visual color of the EEG traces 318f (in the first view area 305a) based on distances of the associated electrode contacts 316f (in the second view area 320a) from a viewpoint. Thus, the visual color of the EEG traces 318f changes, along a predefined grayscale or color-scale, as the distance of associated contacts 316f progressively changes from the viewpoint.

Similarly, in some embodiments, zooming in and out of the 3D brain model 325a, in the second view area 320a, causes the module 125 to automatically adjust the color (grayscale or other color-scales) of the EEG traces 318f, in the first view area 305a, according to the distance of the associated contacts 316f from the viewpoint.

In some embodiments, the multi-modality exam module 125 is configured to automatically create extended bipolar montages. The availability of position information of each electrode and contact allows the module 125 to automatically create one or more extended bipolar montages. Currently clinicians use bipolar montages only within an electrode. In the case of depth electrodes, extending this concept to bipolar montages between contacts from different electrodes may potentially provide additional clinical information. However, the challenge is that with hundreds of available contacts the number of bipolar permutations is overwhelming and, therefore, there is a need to help the clinicians to automatically generate clinically relevant extended bipolar montages.

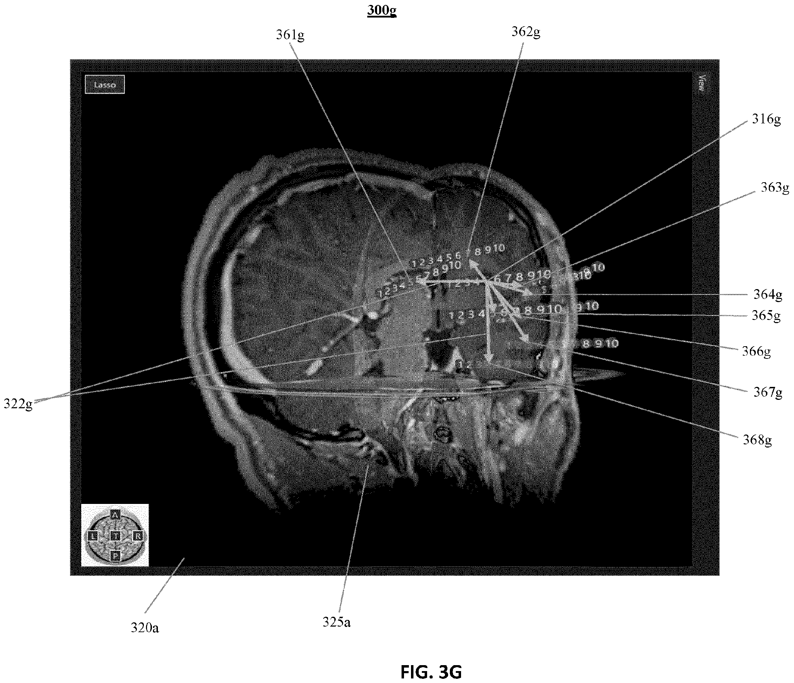

FIG. 3G is a second view area 320a of a unified viewing environment that shows automatic creation of one or more extended bipolar montages, in accordance with some embodiments of the present specification. The module 125 is configured to enable the clinician to easily create an extended bipolar montage based on the "nearest neighbor". In some embodiments, the clinician can select a specific contact 316g and then invoke an extended bipolar montage function such as, for example, by clicking on a related visual graphical element or icon displayed in the unified viewing environment. Invoking the extended bipolar montage function causes the module 125, as it is configured to do so, to automatically generate one or more bipolar montages 322g based on the currently selected contact 316g and the contacts (such as, for example, first contact 361g, second contact 362g, third contact 363g, fourth contact 364g, fifth contact 365g, sixth contact 366g, seventh contact 367g and eighth contact 368g) on each of the other electrodes that are closest in space to the selected contact 316g. It should be appreciated that the first contact 361g, second contact 362g, third contact 363g, fourth contact 364g, fifth contact 365g, sixth contact 366g, seventh contact 367g and eighth contact 368g correspond to different electrodes not only amongst themselves but also in comparison to the electrode related to the selected contact 316g.

In some embodiments, the module 125 supports a bubble feature to auto-select "nearest neighbor" contacts with respect to the selected contact 316g. The clinician may select an icon that provides the clinician with a visual capture tool, such as a bubble, a box, or some other volume defining tool. The clinician uses the volume defining tool to indicate an area of anatomical interest on the 3D brain model 325a. Upon doing so, all electrode contacts in that defined volume are selected to generate one or more montages (including extended bipolar montages).

In some embodiments, the module 125 enables the clinician to visually track what stimulation combinations or montages have already been generated. Thus, if the clinician selects a target electrode contact from the 3D brain model 325a then all other electrode contacts that have already been combined with the target electrode contact are automatically highlighted in the 3D brain model 325a. This provides the clinician with a real-time tracking of previously generated montages. In some embodiments, the module 125 enables the clinician to control the creation of the one or more extended bipolar montages by customizing the maximal distance allowed between the selected contact 316g and the "nearest neighbor" contacts to be used.

In some embodiments, the clinician can make a selection based on EEG signal content that results in bipolar montages. Thus, only bipolar traces with abnormal EEG activity may be allowed to be included in the creation of the one or more montages. In some embodiments, the clinician may manually select EEG traces with abnormal activity thereby highlighting the associated contacts in the 3D brain model 325a and allowing only those contacts to be included in creation of the one or more montages. In some embodiments, the module 125 is configured to automatically select contacts associated with EEG traces showing abnormal activity for the creation of the one or more montages. The clinician is further allowed to manually override such automatic selection of contacts. In some embodiments, the module 125 is configured to automatically ignore low quality contacts for the creation of the one or more montages.

In some embodiments, the multi-modality exam module 125 is configured to support the clinician in the visualization of a plurality of qEEG (quantitative electroencephalography) activities. qEEG is a statistical analysis technique that uses mathematical algorithms to process EEG signals. It can be used to perform a plurality of quantitative analysis of EEG data such as, but not limited to, the analysis of the frequency bands and complexity of signals, the analysis of connectivity and networks, the quantification of the relative power of different physiological EEG frequencies, and the localization of the CNS location of different physiological EEG frequencies, among others. QEEG can be used to help diagnose a variety of conditions, such as, for example: epilepsy, stroke, dementia, traumatic brain injury, mental health disorders, and neuropsychiatric disorders.

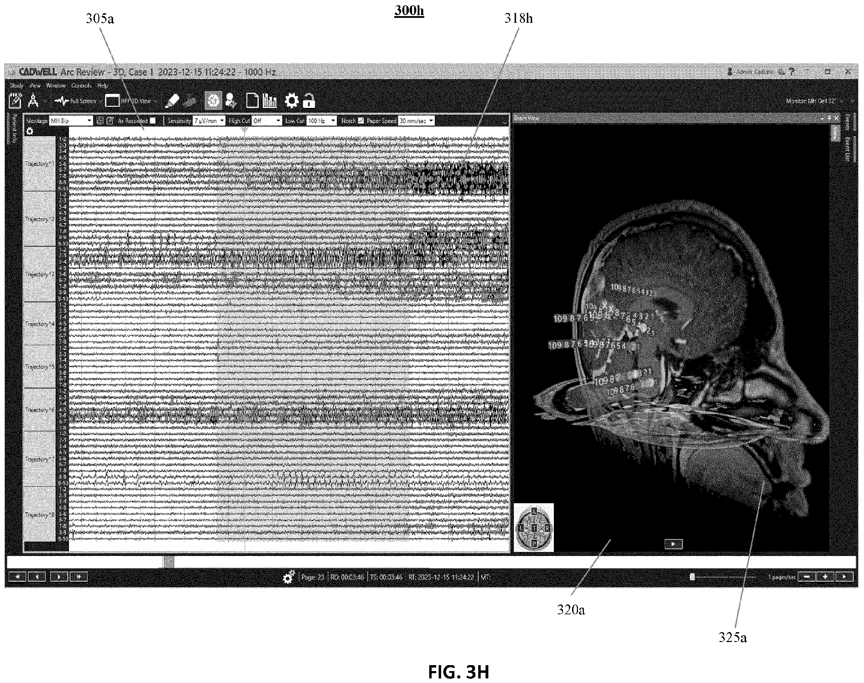

FIG. 3H is a unified viewing environment 300h that illustrates visualization of different aspects and quantitative properties of an EEG in the 3D brain model 325a, in accordance with some embodiments of the present specification. The module 125 is configured such that the clinician can select a point in time and/or a sequence in the EEG data 318h (in the first view area 305a) along with the type of quantitative analysis that they are interested in such as, for example, power of the EEG, frequency content, among other metrics. Thus, the clinician may focus on a single time point within the EEG data 318h and/or choose to play propagation of the EEG data 318h for visualization as image sequences/movies in the second view area 320a. Subsequently, the quantitative properties or results, in which the clinician is interested, are overlaid in the 3D images or brain model 325a using different imaging techniques such as, for instance, color scale.

In some embodiments, the multi-modality exam module 125 is configured to allow the clinician to control and visualize functional brain mapping and testing. Functional brain mapping may be conducted to recognize eloquent areas in the brain by applying electrical stimulation between selected electrode contacts chosen from the recording electrodes. The resulting EEG responses together with the impact it has on the patient, for instance motor twitches, sensory phenomena, and visual impact, among others, are observed and annotated. Significant responses include motor, sensory, visual, and perception as well as spreading brain activation detected by other contacts which can be normal, epilepsy-related, or spontaneous. Each of these categories are manually noted for each contact.

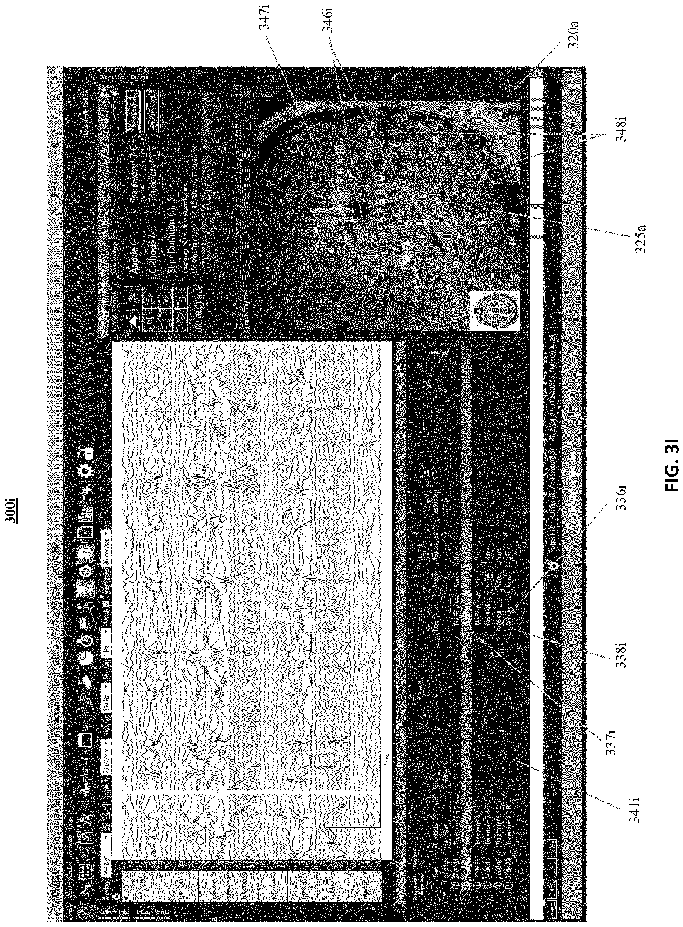

FIG. 3I is a unified viewing environment 300i that shows functional result highlighting, in accordance with some embodiments of the present specification. The module 125 is configured to allow the clinician to select one or more contacts, to be used for stimulation, directly in the 3D brain model 325a (in the second view area 320a) thereby providing the clinician anatomical information regarding the part of the brain that is intended to be stimulated.

The module 125 is configured to automatically indicate, in the 3D brain model 325a, the contacts that have already been stimulated. The module 125 is also configured to automatically indicate, in the 3D brain model 325a, low quality contacts unsuitable for stimulation. The indications may be color coded for ease of visualization.

In some embodiments, functional responses from the stimulation (comprising intracranial electrical stimulation as well as other forms of stimulation such as auditory or visual stimulation) are captured as events with the possibility of assigning them different types of functional impact as well as showing their anatomical location in the 3D brain model 325a. For example, a first functional response is identified as a motor event 336i, a second functional response is identified as a speech event 337i, and a third functional response is identified as a sensory event 338i. The motor event 336i is coded in a first color and has an associated first anatomical location 346i, the speech event 337i is coded in a second color and has an associated second anatomical location 347i while the sensory event 338i is coded in a third color and has an associated third anatomical location 348i.

In some embodiments, the module 125 is configured to allow the clinician to filter the functional responses (in a view area 341i) based at least on their types, for example, with the anatomical indication changing, in the second view area 320a, according to the filtered response type.

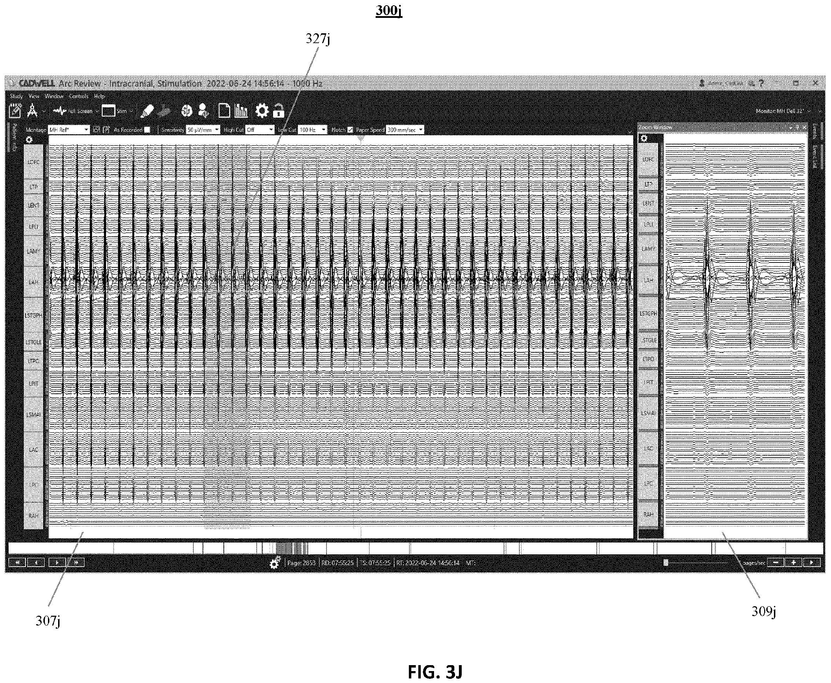

In some embodiments, the multi-modality exam module 125 is configured to support intracranial stimulation contact position validation. The unified environment allows for the visual or automatic confirmation of stimulation electrode placements, and further interrogation of post stimulation volume and pathway conduction can further validate the correct placement of stimulation electrodes. FIG. 3J depicts a viewing environment 300j that shows validation of contact positions for various applications using the information gathered during intracranial stimulation, in accordance with some embodiments of the present specification. FIG. 3J depicts an example of using the software in 2D to be able to zoom in on trace sections and view them in an expanded section alongside on-expanded sections. Referring to FIG. 3J, an expanded view of an artifact is depicted. The viewing environments depicted in FIG. 3I (for the 3D) and FIG. 3J allow a clinician to validate the artifact spread based on electrode locations, and thus get a feel for whether the volume conduction is as expected. A badly placed electrode would not provide the expect conduction. By using the 3D contact position information, in the 3D brain model, and by analyzing the volume conducted artifact from the intracranial stimulation the contact positions can be validated by measuring the actual artifact and comparing it with the expected artifact calculated from the contact positions. A view area 307j shows an artifact waveform 327j for a contact position while another view area 309j displays a zoomed-in portion of the artifact waveform 327j.

The module 125 automatically highlights the contacts (within the 3D brain model) inconsistent with expected values and the clinician is notified to confirm or adjust their locations.

### 3D guided montage generation

In accordance with some embodiments, the multi-modality exam module 125 is configured to support 3D-guided montage generation by allowing the clinician to navigate in the 3D domain (that is, using the 3D brain model displayed in a unified viewing environment) to identify and select anatomically related electrodes and based on those selections, auto-generate one or more electrode montages for simultaneous viewing in the first view area 305a (2D paradigm) and the second view area 320a (3D paradigm).

The association of each EEG trace/channel with the visual representation of the corresponding electrodes and contacts in the patient's 3D brain model allows for ROI (region of interest) based montages. Traditionally the montages used to derive EEG data are either created using a common reference contact (referential montages) or by creating montages looking at activity between adjacent contacts in the same electrode (bipolar montages). In contrast, the ROI montages are created based on an anatomical region or location and the contacts that are in the vicinity of that location.

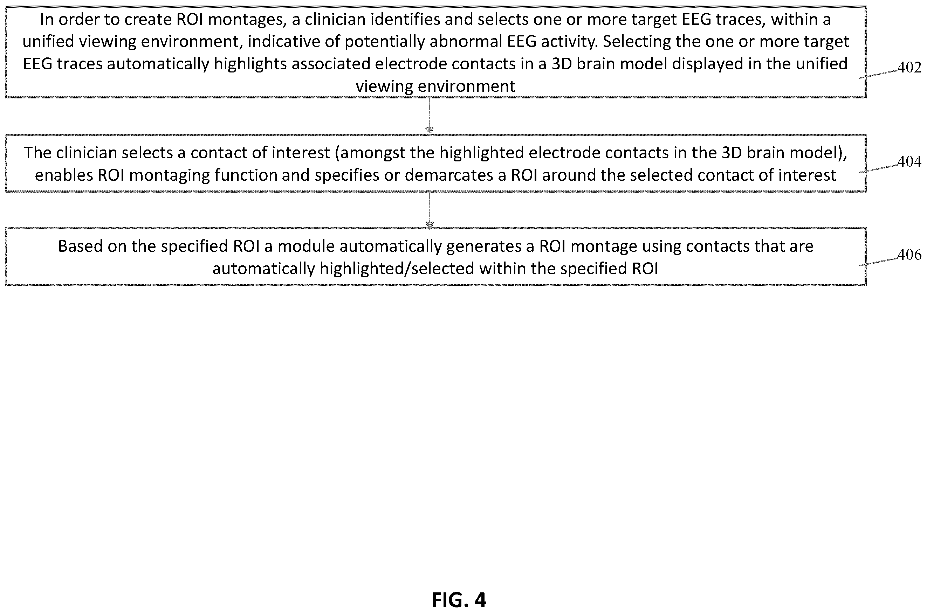

FIG. 4 is a flowchart of a plurality of exemplary steps of a method of creating ROI montages, in accordance with some embodiments of the present specification. In embodiments, the module 125 is configured by executing a plurality of instructions of programmatic code, to support the methodology used for the creation of ROI montages.

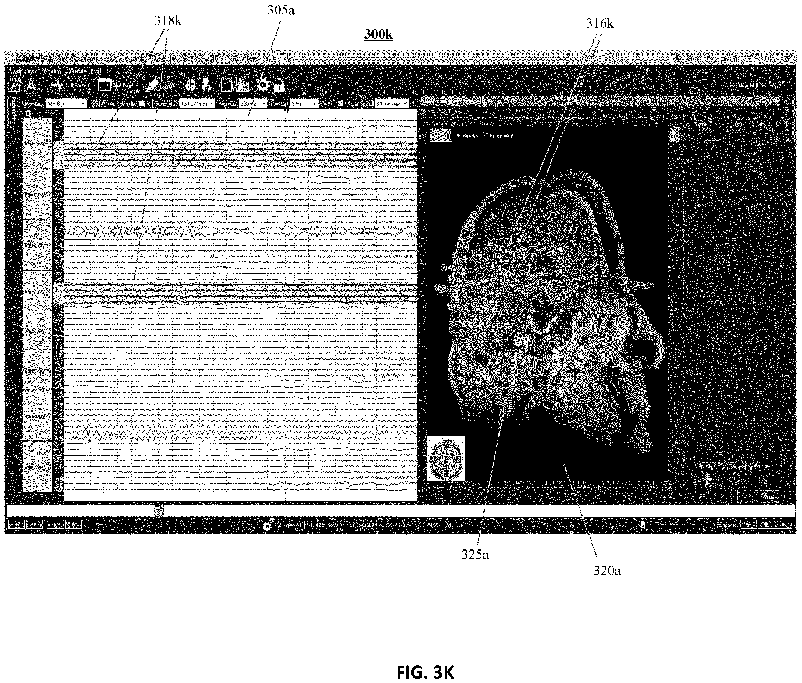

At step 402, in order to create ROI montages, the clinician identifies and selects one or more target EEG traces, within a unified viewing environment, indicative of potentially abnormal EEG activity. Selecting the one or more target EEG traces automatically highlights the associated one or more electrode contacts in the 3D brain model displayed in the unified viewing environment. FIG. 3K is a unified viewing environment 300k that shows EEG traces and associated electrode contacts highlighted, in accordance with some embodiments of the present specification. In response to the clinician's selection of EEG traces 318k (indicative of potentially abnormal EEG activity), in the first view area 305a, the associated electrode contacts 316k are automatically highlighted (for example, using a predefined color code and/or text-based annotation) in the 3D brain model 325a displayed in the second view area 320a.

At step 404, the clinician selects a contact of interest (amongst the highlighted electrode contacts in the 3D brain model), enables the ROI montaging function (for example, by clicking on a graphical viewing element or icon displayed in the unified viewing environment) and specifies or demarcates a ROI around the selected contact of interest. In some embodiments, once selected, the contact of interest may be highlighted in a predefined color.

In some embodiments, upon enabling the ROI montaging function, the configured module 125 provides the clinician with a ROI or volume capture tool to enable the clinician to manually create (expand/collapse) a 3D geometrical shape (such as, for example, a sphere) around the contact of interest in order to auto-select other contacts which fall within the 3D geometrical shape. The size and location of the geometrical shape is indicative of the ROI specified by the clinician and hence indicative of the auto-selection of other contacts that fall within the geometrical shape. The module 125 is configured to automatically highlight any contact inside the geometrical shape, no matter what electrode it belongs to, together with any EEG data recorded from the highlighted contacts. That is, the highlighted contacts may not necessarily be related to a single electrode.

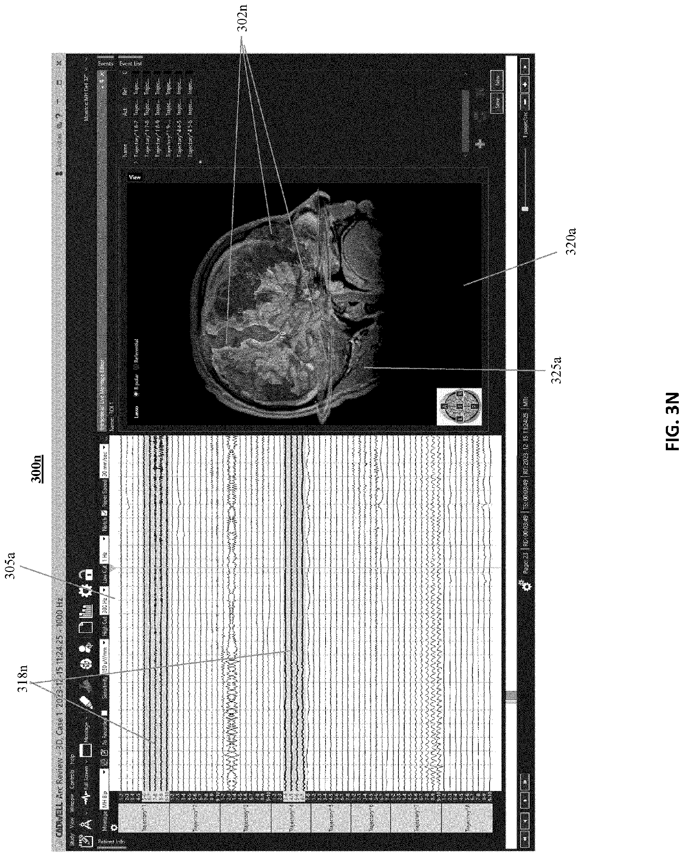

Alternatively or additionally, in some embodiments, upon enabling the ROI montaging function, the module 125 is configured to provide a predefined anatomical atlas in order to enable the clinician to choose a ROI from the anatomical atlas. In some embodiments, the anatomical atlas is available for visualization within the 3D brain model. FIG. 3N is a unified viewing environment 300n that shows an anatomical atlas within the 3D brain model 325a, in accordance with some embodiments of the present specification. As shown, the anatomical atlas shows various anatomical regions 302n that may be color-coded, using a predefined color-scale, within the 3D brain model 325a (in the second view area 320a). In response to the clinician selecting one of the various anatomical regions 302n, all electrode contacts 318n within the selected one of the various anatomical regions 302n are automatically highlighted, in the first view area 305a, no matter what electrodes they belong to, together with any EEG data recorded from the highlighted contacts. In some embodiments, individual contacts can be included or excluded to customize the creation of a montage to further examine and define a ROI. Thus, these contacts could be spread across different electrodes and are able to be grouped together geographically in a unique way due to the underlying knowledge of their relation to each other in the specified or demarcated ROI or 3D anatomical

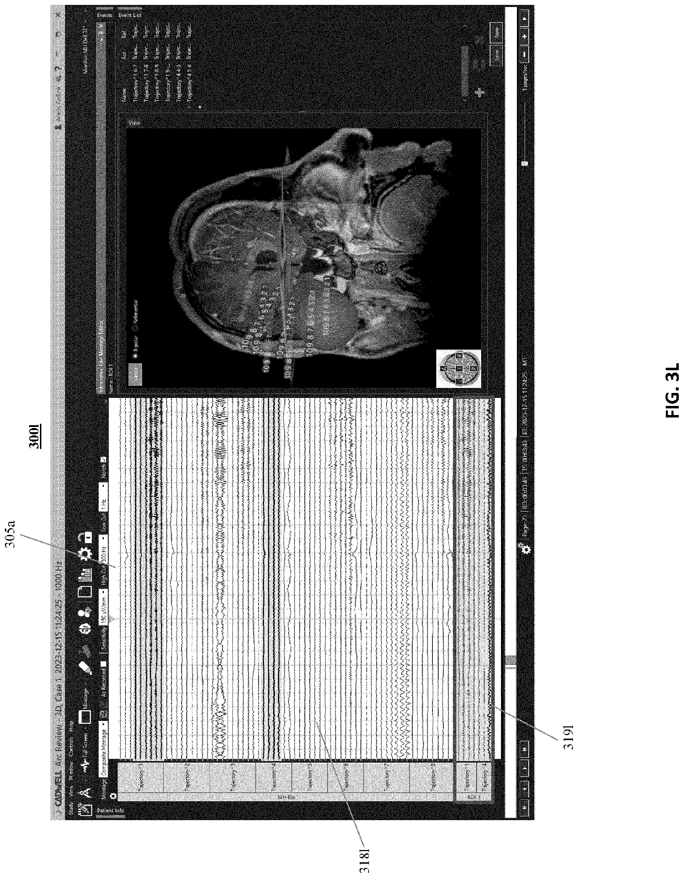

At step 406, based on the specified ROI, the module 125 automatically generates a ROI montage using contacts that are automatically highlighted/selected within the specified ROI. In various embodiments, the module 125 automatically creates different types of montages such as, for example, referential, bipolar, and extended bipolar. FIG. 3L is a unified viewing environment 300l that shows an ROI montage displayed in the first view area 305a, in accordance with some embodiments of the present specification. As shown, in some embodiments, the created ROI montage 319l is displayed at the bottom of the existing vertical stack of EEG traces 318l. In alternate embodiments, the created ROI 319l montage may be displayed at the top, left side or right side of the existing vertical stack of EEG traces 318l.

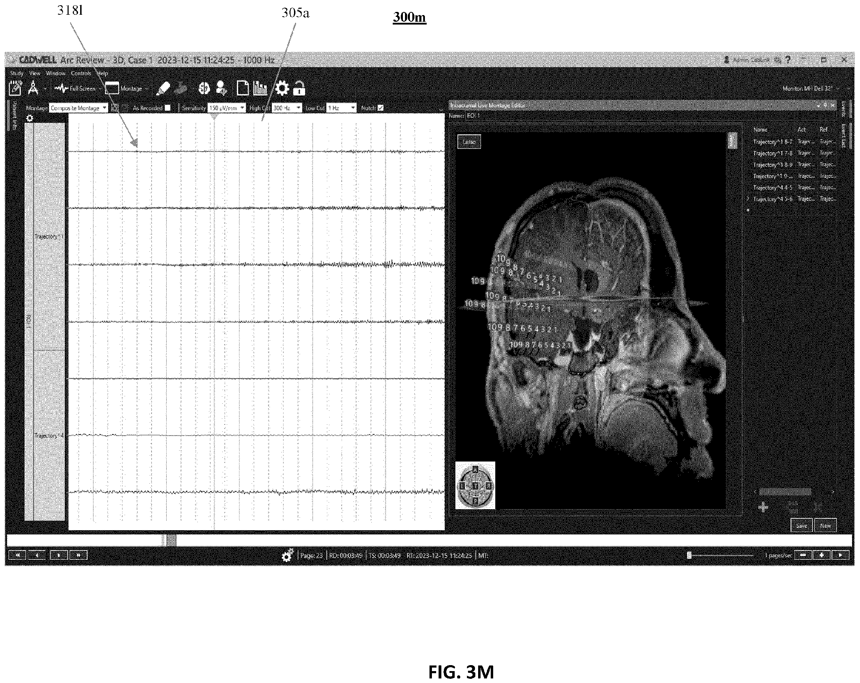

In some embodiments, the module 125 is configured to allow the clinician to select and view only the newly created ROI montage 318l in the first view area 305a. FIG. 3M is a unified viewing environment 300m that shows only the newly created ROI montage 318l in the first view area 305a, in accordance with some embodiments of the present specification. The clinician may, for example, click (select) over the ROI 319l (shown in FIG. 3L) displayed at the bottom of the existing vertical stack of EEG traces 318l, in the unified viewing environment 300l of FIG. 3L, in order to show only the newly created ROI montage 318l in the first view area 305a - as shown in the unified viewing environment 300m.

### Identification of potentially noisy/bad (i.e. low or poor quality) channels for manual or automatic exclusion

Poor quality electrodes do not reliably represent the patient's brain activity. There are many reasons for poor quality including, but not limited to the following situations: a contact may not be well-connected to the patient's brain (high impedance), a contact may not be located in neuronal tissue whose primary function is information signaling (gray matter), a contact may not be located in the patient's brain, and/or the contact's electrical activity is statistically too different (indicative of, for example, too low signal to noise ratio) from neighboring electrodes.

In embodiments, the association of each EEG trace/channel with the visual representation of the corresponding electrodes and contacts in the patient's 3D brain model allows for a novel method of assessing contact quality and data validity. The module 125, using the association, is configured to automatically and accurately identify and highlight potentially noisy/bad channels (and associated electrodes and contacts) based at least on knowing where those electrodes are located in the brain. Alternatively, or additionally, the module 125 is configured to allow the clinician to manually identify and select noisy/bad channels. Accordingly, the module 125 is configured to either automatically exclude or enable the clinician to manually exclude the detected noisy/bad channels.

In some embodiments, the module 125 is configured to automatically detect potentially bad channels using machine learning of a plurality of EEG signal features. The plurality of features quantify variance, spatial-temporal correlation and nonlinear properties of EEG signals and, in some embodiments, include correlation coefficient, variance, deviation, amplitude, gradient, Hurst exponent and Kurtosis. Since the number of bad channels is usually much lower than the number of good channels, in some embodiments, the module 125 implements an ensemble bagging machine learning/classifier model.

The method of automatically detecting potentially bad channels includes a first step of training the classifier model. In some embodiments, the classifier model is trained using training data indicative of the plurality of features from stored EEG datasets where bad channels are previously labeled by experts. Post training of the classification model, at a second step, the plurality of features are extracted from new EEG datasets and provided as input to the trained classification model, at a third step, in order for the trained classification model to predict bad channels.

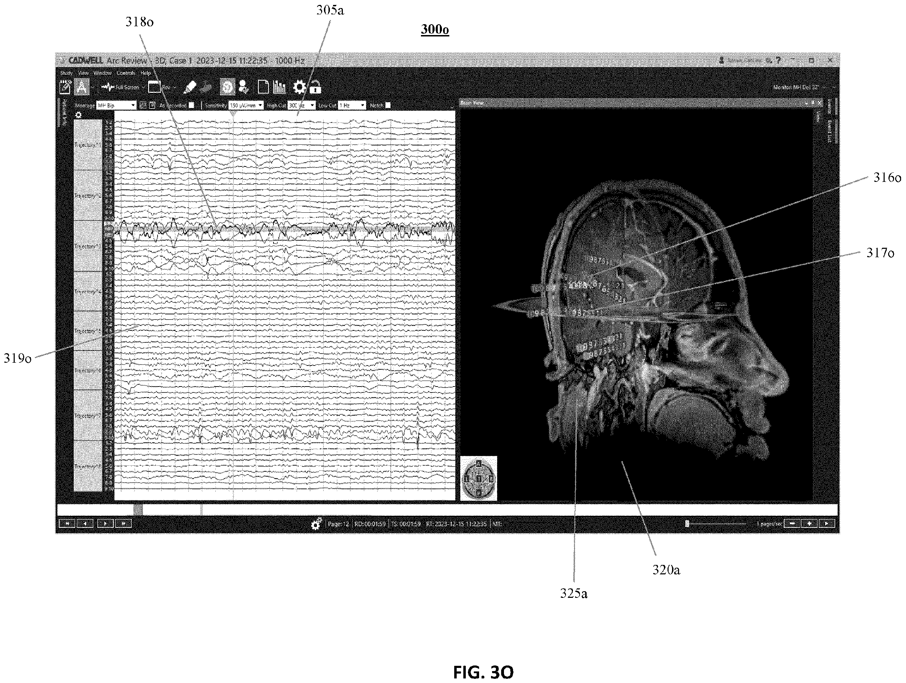

FIG. 3O is a unified viewing environment 300l showing a visual indication of contact quality in the displayed 3D brain model as well as in the EEG traces, in accordance with some embodiments of the present specification. As shown, the quality of electrodes and contacts as well as the associated EEG traces is visually indicated using a predefined color-scale. For example, low quality contacts 316o are highlighted in a first color, such as red in the 3D brain model 325a displayed in the second view area 320a. The associated low quality EEG traces 318o are also shown in a first color in the first view area 305a. Good quality contacts 317o are highlighted in a second color, such as green, in the 3D brain model 325a while the associated good quality EEG traces 319o are not highlighted in any color conveying their being good quality. Alternatively, the associated good quality EEG traces 319o may also be highlighted in a second color.

Additional color codes may be used to identify different categories of poor-quality electrodes and contacts. That is, a contact (and associated EEG trace) detected as being not well connected to the patient's brain (high impedance) may be highlighted in a third color, a contact (and associated EEG trace) detected as being not located in neuronal tissue whose primary function is information signaling (gray matter) may be highlighted in a fourth color, a contact (and associated EEG trace) detected as being not located in the patient's brain may be highlighted in a fifth color, and a contact (and associated EEG trace) detected as having electrical activity statistically too different (indicative of, for example, too low signal to noise ratio) from neighboring electrodes may be highlighted in a sixth color.

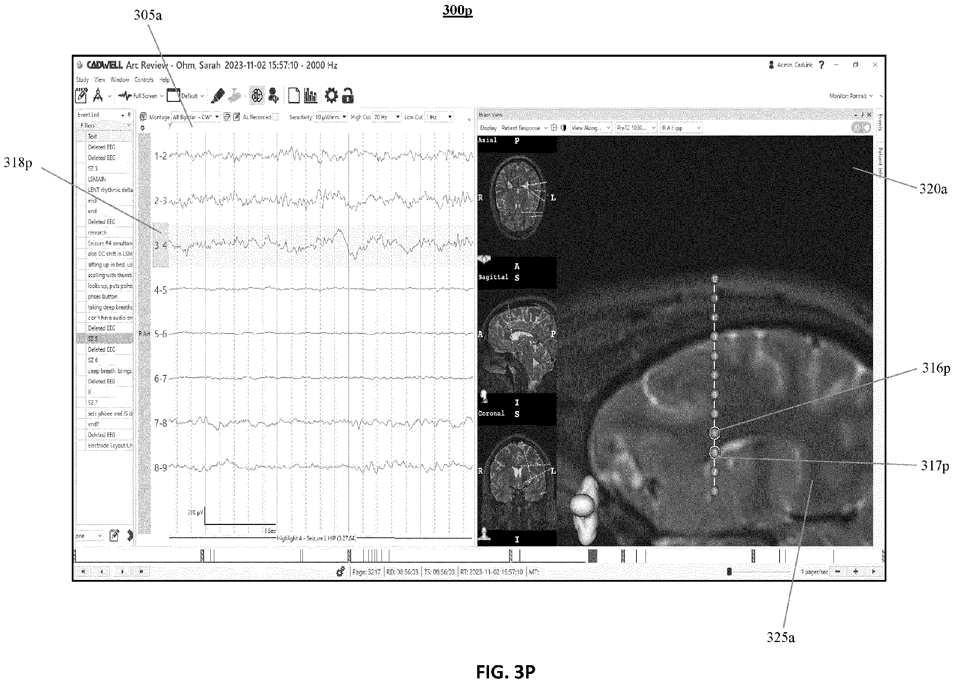

FIG. 3P shows a unified viewing environment 300p in which detected visual indication of contact quality in the displayed 3D brain model as well as in the EEG traces is shown, in accordance with some embodiments of the present specification. As shown, the EEG trace 318p (in the first view area 305a) and associated contacts 316p and 317p, displayed in the 3D brain model 325a (in the second view area 320a) are automatically highlighted by the module 125. The highlighting signifies that the contacts 316p and 317p have electrical activity statistically too different from neighboring electrodes (based on the module 125 performing statistical analyses of the EEG traces).

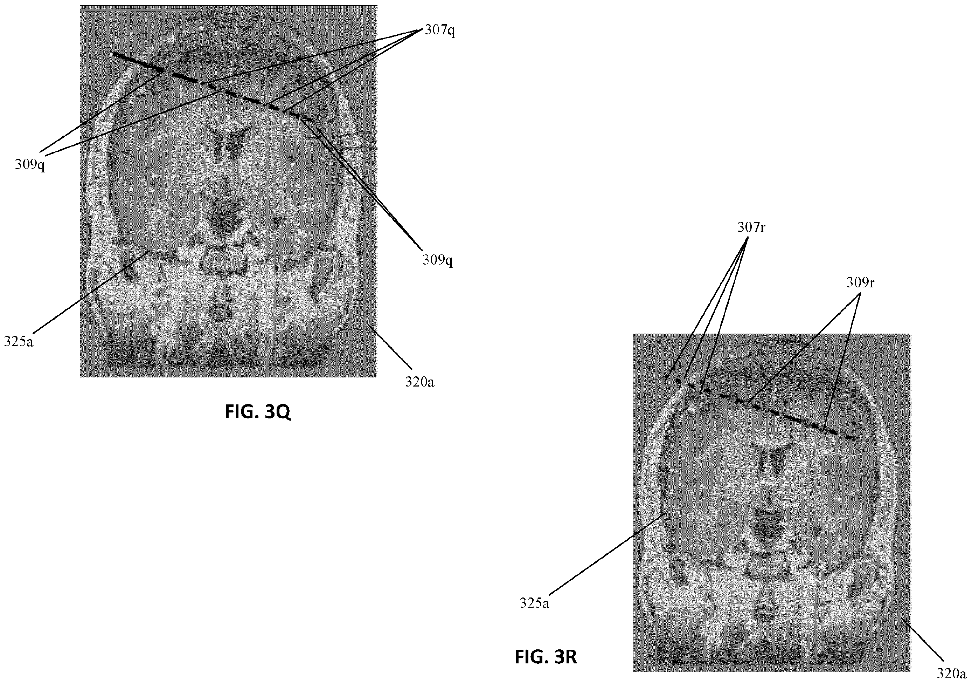

FIG. 3Q shows the detection of quality of contacts based on their spatial location within a patient's brain, in accordance with some embodiments of the present specification. As shown in the 3D brain model 325a displayed in the second view area 320a, contacts on electrodes are distributed between gray and white matter. To reduce displayed channel count, the contacts 307q positioned in white matter are identified (and highlighted in a first color, in the 3D brain model 325a) and removed from EEG recording whereas the contacts 309q positioned in gray matter are identified (and highlighted in a second color) and retained. That is, contacts positioned outside of the gray matter are automatically detected and excluded.

FIG. 3R shows the detection of quality of contacts based on their spatial location within and outside a patient's brain, in accordance with some embodiments of the present specification. As shown in the 3D brain model 325a displayed in the second view area 320a, contacts on electrodes are distributed both in and outside the patient's brain. To reduce displayed channel count, the contacts 307r positioned outside the brain are identified (and highlighted in a first color, in the 3D brain model 325a) and removed from EEG recording whereas the contacts 309r positioned in the brain are identified (and highlighted in a second color) and retained. That is, contacts positioned outside of the patient's brain are automatically detected and excluded.

### Pop-up Displays

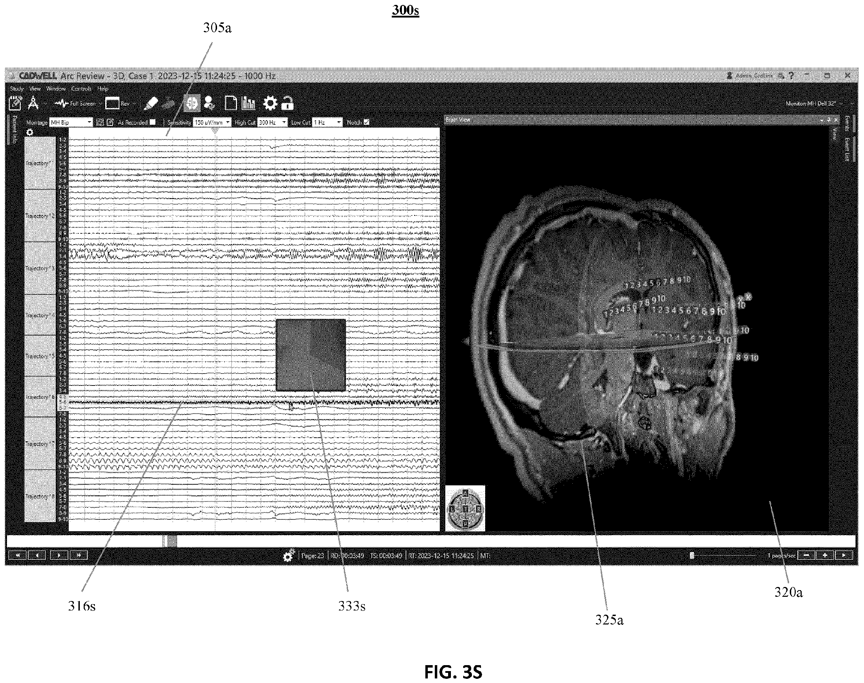

In some embodiments, the module 125 is configured to enable the clinician to view, within a pop-up display window, an anatomy associated with an EEG trace and vice versa. FIG. 3S is a unified viewing environment 300s that shows an anatomy pop-up window displayed over an associated EEG trace, in accordance with some embodiments of the present specification. When the clinician points, hovers or clicks the mouse over an EEG trace 316s, in the first view area 305a, the module 125 is configured to automatically display a pop-up window 333s, in the first view area 305a, showing an anatomy centered around the one or more contacts associated with the EEG trace 316s. If multiple electrodes and contacts are used to derive the EEG trace 316s, then the chosen anatomical location will be based on the derivation used and/or user input. An anatomy associated with the position of the chosen electrode is shown in the pop-up window 333s. In some embodiments, the module 125 is configured to enable the clinician to rotate and zoom in/out of the EEG trace 316s within the first view area 305a.

Similarly, when the clinician points, hovers or clicks the mouse over a contact displayed in the 3D brain model 325a, in the second view area 320a, the module 125 (as it is configured to do so) automatically displays associated EEG data (for example, in the form of one or more EEG traces) in a pop-up window in the second view area 320a. This feature is advantageous at least for verifying contacts in white matter.

### Identifying brain regions connectivity and subsequent analysis

Referring back to the unified viewing environment 300a of FIG. 3A, in some embodiments, the module 125 is configured to use the association of each EEG trace/channel with the visual representation of the corresponding electrodes and contacts in the patient's 3D brain model to automatically or manually enable the clinician to visually annotate, mark or highlight connectivity between two or more brain regions onto the 3D brain model 325a in the second view area 320a and/or onto the EEG traces (associated with the connected brain regions) in the first view area 305a.

Additionally, in some embodiments, the module 125 is configured to automatically or manually enable the clinician to visually annotate, mark or highlight one or more results of brain connectivity analysis, performed by the clinician on the connected brain regions, onto the 3D brain model 325a in the second view area 320a and/or onto the EEG traces (associated with the connected brain regions) in the first view area 305a.

In the second view area 305a, connectivity between two or more brain regions may by visually represented, in some embodiments, with a 'line' connecting the brain regions in the 3D brain model 325a (3D paradigm). Alternatively, or additionally, connectivity between two or more brain regions may be visually represented with a change in color of the brain regions and/or electrodes.

In the first view area 305a, connectivity between two or more brain regions may by visually represented, in some embodiments, by highlighting the EEG traces (2D paradigm) associated with the connected brain regions.

In some embodiments, if the clinician uses the mouse to hover over, point at or click on the 'line' indicative of connectivity between two or more brain regions in the 3D brain model 325a (in the second view area 320a), the module 125 is configured to automatically highlight the EEG traces (in the first view area 305a) associated with the electrodes and contacts in the connected two or more brain regions. Similarly, in some embodiments, if the clinician uses the mouse to hover over, point at or click on the highlighted EEG traces (in the first view area 305a) associated with the connected two or more brain regions, the module 125 is configured to automatically highlight (or indicate by a change in color) the connected two or more regions in the 3D brain model 325a (in the second view area 320a).

In some embodiments, the module 125 is configured to enable the clinician to montage the connected two or more brain regions alone for quick analyses.

Thus, the visual annotation, mark or highlighting enables the clinician to identify connected brain regions for further analyses. Users performing the connectivity analysis for either research or to establish connected brain regions for clinical purposes (such as for safe surgical resection), can do so within a precision inherited from the pre-surgical data.

### Other functions

Additionally, in various embodiments, the module 125 is further configured to:
a) Generate an operative report with tests performed and results observed, movie or still images of responses and analytics. The clinician may also want to review the procedure again prior to surgical ablation and resection.
b) Record an entire electrodes placement surgical procedure for teaching and research purposes.
c) Indicate which contacts have to be mapped to specific inputs on the amplifier (for example, the contacts are green after mapping and gray prior to mapping).

The above examples are merely illustrative of the many applications of the systems and methods of the present specification. Although only a few embodiments of the present invention have been described herein, it should be understood that the present invention might be embodied in many other specific forms without departing from the spirit or scope of the invention. Therefore, the present examples and embodiments are to be considered as illustrative and not restrictive, and the invention may be modified within the scope of the appended claims.

## Claims

1. A computer-implemented method of enabling a user to visualize EEG data in combination with pre-surgical data and post-surgical data using a computing device, wherein the computing device comprises at least one processor configured to execute programmatic instructions and a non-transient memory adapted to store the programmatic instructions, comprising:
generating a first graphical user interface, wherein the first graphical user interface comprises at least a first display area and a second display area, wherein the first display area is separate and distinct from the second display area, and wherein the first and second display areas are positioned side-by-side;
acquiring, using the computing device, the pre-surgical data and the post-surgical data, wherein the pre-surgical data comprises a three-dimensional first image of a patient's brain and comprises data indicative of a visual representation of at least one of an electrode trajectory and an electrode profile, wherein the post-surgical data comprises a three-dimensional second image of the patient's brain and comprises data indicative of a visual representation of at least one electrode surgically placed in the patient's brain, and wherein the three-dimensional first image and the three-dimensional second image are co-registered with a same three-dimensional coordinate system;
acquiring the EEG data of the patient;
displaying, simultaneously, the EEG data in the first display area and the post-surgical data in the second display area of the first graphical user interface, wherein the EEG data is displayed as a plurality of EEG traces; and
automatically visually associating the visual representation of each of the at least one electrode in the post-surgical data with a corresponding one of the plurality of EEG traces.

2. The computer-implemented method of claim 1, further comprising enabling a comparison of the post-surgical data with the pre-surgical data by highlighting, in the post-surgical data displayed in the second display area, one or more of the at least one electrode that deviates from the electrode trajectory by more than a predefined amount.

3. The computer-implemented method of claim 2, wherein the predefined amount is an offset percentage and wherein the offset percentage is in a range of 1% to 75%.

4. The computer-implemented method of any preceding claim, wherein the three-dimensional first image and the three-dimensional second image are at least one of a MRI image, a CT image, a SPECT image or a PET image.

5. The computer-implemented method of any preceding claim, wherein the at least one electrode comprises a plurality of electrodes and wherein the method further comprises:
providing the user with a second graphical user interface adapted to receive a selection of one or more first contacts from a first one of the plurality of electrodes and a selection of one or more second contacts from a second one of the plurality of electrodes, wherein the first one of the plurality of electrodes and the second one of the plurality of electrodes are positioned respectively in a first anatomical region of the patient's brain and a second anatomical regions of the patient's brain and wherein the first anatomical region and the second anatomical region are different; and
grouping said one or more first contacts and the one or more second contacts.

6. The computer-implemented method of any preceding claim, further comprising:
providing the user with a second graphical user interface adapted to receive a selection of one or more EEG traces of the plurality of EEG traces, wherein the selected one or more EEG traces are indicative of the patient's potentially abnormal EEG activity;
automatically highlighting, within the three-dimensional second image, one or more contacts of the at least one electrode that is associated with the selected one or more EEG traces;
providing the user with a third graphical user interface adapted to receive a selection of at least one of the one or more contacts;
providing the user with a fourth graphical user interface adapted to receive a specification of a region of interest around the selected at least one of the one or more contacts, wherein contacts of the at least one electrode that fall within the specified region of interest are automatically highlighted; and
automatically generating an EEG montage of the region of interest using the contacts that are automatically highlighted within the specified region of interest.

7. The computer-implemented method of claim 6, wherein the highlighted contacts within the specified region of interest are physically associated with more than one electrodes of the at least one electrode.

8. The computer-implemented method of claim 6 or 7, wherein the fourth graphical user interface is adapted to receive a geometrical shape, drawn by the user, around the selected electrode contact to specify the region of interest.

9. The computer-implemented method of any one of claims 6 to 8, wherein the fourth graphical user interface is adapted to retrieve data from an anatomical database to specify the region of interest.

10. The computer-implemented method of any preceding claim, further comprising:
automatically identifying inoperable channels; and
automatically highlighting the identified inoperable channels in the three-dimensional second image.

11. The computer-implemented method of claim 10, further comprising automatically highlighting in the first display area one or more EEG traces of the plurality of EEG traces associated with the identified inoperable channels.

12. The computer-implemented method of claim 10 or 11, wherein the identified inoperable channels are associated with one or more of the at least one electrode positioned within white matter of the patient's brain.

13. The computer-implemented method of claim 10 or 11, wherein the identified inoperable channels are associated with one or more of the at least one electrode located outside the patient's brain.

14. The computer-implemented method of any preceding claim, wherein the graphical user interface is configured to automatically generate a pop-up graphical user interface window when the user clicks an EEG trace of the plurality of EEG traces or causes a cursor to hover over said EEG trace, and wherein the pop-up graphical user interface window is configured to display the patient's brain anatomy that is centered proximate the at least one electrode associated with said EEG trace.

15. The computer-implemented method of any preceding claim, wherein the graphical user interface is configured to automatically generate a pop-up graphical user interface window when the user clicks a contact of the at least one electrode or causes a cursor to hover over said contact, and wherein the pop-up graphical user interface window is configured to display an EEG trace of the plurality of EEG traces associated with said contact.

16. The computer-implemented method of any preceding claim, further comprising:
automatically generating data indicative of a degree of connectivity between two or more regions in the three-dimensional second image; and,
visually annotating said data indicative of the degree of connectivity in the three-dimensional second image.

17. The computer-implemented method of claim 16, wherein the visual annotation comprises one or more lines connecting the two or more regions.

18. The computer-implemented method of claim 16, wherein the visual annotation comprises a color, different from a remainder of the three-dimensional second image, indicative of the degree of connectivity between the two or more regions.

19. The computer-implemented method of any one of claims 16 to 18, further comprising:
automatically highlighting one or more EEG traces of the plurality of EEG traces associated with the two or more regions.

20. The computer-implemented method of any preceding claim, further comprising:
generating a second graphical user interface adapted to receive a selection of an anatomical space in the three-dimensional second image; and
creating a label related to the anatomical space.

21. A computer program which, when executed on at least one processor of a computing device, is configured to carry out the method of any preceding claim.
